# EUROPÄISCHE PATENTSCHRIFT

(11) **EP 2 931 375 B1**
(45) Veröffentlichungstag und Bekanntmachung des Hinweises auf die Patenterteilung: **18.09.2019**
(21) Anmeldenummer: 13826993.1
(22) Anmeldetag: 11.12.2013
(51) Int. Cl.: A61P 35/00, A61K 38/19, A61K 38/21

(54) **VERWENDUNG VON WIRKSTOFFKOMBINATIONEN ZUR INDUKTION EINER TUMORSENESZENZ**
USE OF ACTIVE SUBSTANCE COMBINATIONS FOR INDUCING TUMOUR SENESCENCE
UTILISATION DE COMBINAISONS DE SUBSTANCES ACTIVES POUR INDUIRE UNE SÉNESCENCE TUMORALE

(30) Priorität: 11.12.2012 DE 102012024749
(43) Veröffentlichungstag der Anmeldung: 21.10.2015
(73) Patentinhaber: Röcken, Martin, 72076 Tübingen (DE)
(72) Erfinder: WIEDER, Thomas, 72144 Dusslingen (DE); HAHN, Matthias, 72076 Tübingen (DE); BRENNER, Ellen, 72070 Tübingen (DE); BRAUMÜLLER, Heidi, 72074 Tübingen (DE); BRAUNGART, Kilian, 72070 Tübingen (DE); ASSMANN, Sonja, 73529 Schwäbisch Gmünd (DE); RÖCKEN, Martin, 72076 Tübingen (DE)
(74) Vertreter: Jungblut & Seuss
(86) Internationale Anmeldenummer: PCT/DE2013/000794
(87) Internationale Veröffentlichungsnummer: WO 2014/090224

(56) Entgegenhaltungen:
- EP-A1- 0 170 843
- WO-A1-2010/132867
- WO-A2-2005/007086
- US-A- 4 650 674
- US-A1- 2012 258 117
- E CRESCENZI ET AL: "NF-[kappa]B-dependent cytokine secretion controls Fas expression on chemotherapy-induced premature senescent tumor cells", ONCOGENE, Bd. 30, Nr. 24, 16. Juni 2011 (2011-06-16) , Seiten 2707-2717, XP55004211, ISSN: 0950-9232, DOI: 10.1038/onc.2011.1
- R. L. MARQUET ET AL: "Anti-tumor activity of recombinant mouse tumor necrosis factor (TNF) on colon cancer in rats is promoted by recombinant rat interferon gamma; toxicity is reduced by indomethacin", INTERNATIONAL JOURNAL OF CANCER, Bd. 40, Nr. 4, 15. Oktober 1987 (1987-10-15), Seiten 550-553, XP55110266, ISSN: 0020-7136, DOI: 10.1002/ijc.2910400419
- BRAUMUELLER HEIDI ET AL: "T-helper-1-cell cytokines drive cancer into senescence", NATURE (LONDON), Bd. 494, Nr. 7437, Februar 2013 (2013-02), Seiten 361-365, XP002722482, ISSN: 0028-0836
- VIJAYA CHATURVEDI ET AL: "Role of INK4a/Arf Locus-Encoded Senescent Checkpoints Activated in Normal and Psoriatic Keratinocytes", THE AMERICAN JOURNAL OF PATHOLOGY, Bd. 162, Nr. 1, 1. Januar 2003 (2003-01-01), Seiten 161-170, XP55110634, ISSN: 0002-9440, DOI: 10.1016/S0002-9440(10)63807-2
- 43rd annual meeting of the arbeitsgemeinschaft dermatologische Forschung; P227; Interferon-alpha-based immunotherapy induces senesce in human cancer cells in vivo; T.Wieder et al.

## Beschreibung

Die vorliegende Erfindung betrifft eine Kombination von Substanzen und deren Verwendung zur Tumorbehandlung durch Induktion einer Tumorseneszenz.

Eine erfolgreiche Tumorprävention und -therapie bleibt nach wie vor trotz der vielen verschiedenen Ansätze der vergangenen Jahrzehnte eine große Herausforderung in der Wissenschaft und der Medizin. In der Regel erfolgen heutzutage Tumortherapien durch eine operative Tumorentfernung, mittels Bestrahlung, wobei hier in der Regel ionisierende Strahlung zum Einsatz kommen, und/oder eine Chemotherapie. Die Therapien werden hierzu auch kombiniert. Neueste Therapien basieren auf der Interaktion von Medikamenten mit Signalkaskaden die Tumoren zu einer krankhaften Vermehrung verhelfen. Wichtig ist, dass alle bisher entwickelten Therapien i.d.R. nur während der Therapieapplikation wirken. Verschiedene Daten legen nahe, dass keine der bisher zur Verfügung stehenden Therapien es schafft Tumoren ganz zu eradizieren. Aus diesem Grunde neigen Tumoren dazu, vermutlich von Tumorstammzellen ausgehend, zu rezidivieren (Ref. Nature 2012).

Ferner wurden Krebsimmuntherapien entwickelt, im Rahmen derer der betroffene Patient mit für den Tumor speziellen Oberflächenantigenen geimpft wird, mit dem Ziel, Immunantworten einzuleiten, also die Tumorzellen über zytotoxische CD8 T Lymphozyten, auch 'Killerzellen' genannt, zu töten. Bei wiederum anderen Therapieansätzen werden Immunmodulatoren eingesetzt, mit Hilfe derer das Immunsystem des Patienten so angeregt wird, dass körpereigene Abwehrmechanismen so wieder hergestellt/aktiviert werden, dass sie damit den Tumor zerstören/töten können. Bei diesen Therapien werden durch die Aktivierung der körpereigenen oder - im Falle der allogenen Knochenmarkstransplantation - körperfremden Immunantwort die bösartigen Tumorzellen zerstört; dabei erfolgt meist eine unspezifische Aktivierung des Immunsystems, oder der Transfer von Tumor-spezifischen zytotoxischen Lymphozyten. Auch natürliche Killerzellen werden eingesetzt. Auch für diese Anwendungsformen gilt, dass viele der Therapien nur während der Therapieapplikation wirken, denn sie führen in den seltensten Fällen zur vollständigen Tumoreradikation. Auch hier neigen die Tumoren vermutlich von Tumorstammzellen ausgehend zu rezidivieren (Ref. Nature 2012).

Die Rolle des Immunsystems bei der Tumorentwicklung wird in neueren Aufsätzen neu beleuchtet: Unter bestimmten Umständen kann ein Gleichgewichtszustand erreicht werden, so dass die Entwicklung neuer Tumorzellen und die Vernichtung bestehender Tumorzellen temporär zu einem Stillstand des Tumorwachstums führt (Koebel et al., Nature 450, 903 (2007); Schreiber et al., Science 331,1565 (2011)).

Crescenzi et al. (Oncogene (2011) 30, 2707-2717, beschreiben Untersuchungen an seneszenten Tumorzellen, wobei die Seneszenz durch einmalige Exposition mit Doxorubicin induziert wurde.

Vor diesem Hintergrund stellt die effiziente Kontrolle der Tumorstammzellen und die effiziente Inhibierung eines exponentiellen Wachstums der sich daraus entwickelnden Tumorzellen durch körpereigene Abwehrmechanismen eine große Herausforderung für die behandelnden Ärzte dar. Trotz der Induktion starker, körpereigener Abwehrmechanismen, die initial zu einer Tumorreduktion führen, neigen sie meist dazu nach Beendigung der Therapie erneut zu starten und nach Beendigung der Therapie erneut wieder zu wachsen. Ein anderes Problem bringen die Medikamente mit sich, die eine unspezifische Immunaktivierung hervorrufen, da deren Verabreichung mit starken Nebenwirkungen einhergehen. Bis heute geht man davon aus, dass das Anhalten des Tumorwachstums durch das Immunsystem, sofern es eintritt, primär darauf beruht, dass die Immunantwort die Tumorzellzerstörung so beschleunigt, dass ein Gleichgewicht zwischen Tumorzellzerstörung und der natürlichen Tumorzellproliferation besteht.

Vor diesem Hintergrund ist es Aufgabe der vorliegenden Erfindung einen neuen, zu den bisherigen auf Zytotoxizität ausgerichteten Therapieansätzen komplementären Therapieansatz bereitzustellen, mit welchem die Entwicklung, die Entstehung und das Wachstum von Tumoren effektiv behandelt oder gar verhindert werden kann. Diese Therapie beruht darauf, dass das Wachstum und die Vermehrung von Tumorzellen - einschliesslich der Tumorstammzellen - langfristig stillsteht.

Erfindungsgemäß wird diese Aufgabe gelöst durch die Verwendung einer Kombination aus zumindest zwei unterschiedlichen Substanzen, von denen die eine die STAT1-Signalkette aktiviert und die andere die Moleküle der TNFR1-Signalkette oder der CD95 Signalkette aktiviert, zur Behandlung und/oder Prävention von Tumoren durch Induktion eines dauerhaften Wachstumsarrests (Seneszenz), wobei die Substanzen, die einerseits den STAT1-Signalweg und andererseits den TNFR1/CD95-Signalweg aktivieren, miteinander gekoppelt sind oder als einzelne Substanzen verabreichbar sind. Sie dienen insbesondere der Behandlung und Prävention von Tumoren, bei welchen p16^{lnk4a} präsent ist.

Gegenstand der vorliegenden Erfindung ist daher die Verwendung einer Kombination aus zumindest zwei unterschiedlichen Substanzen, von denen die eine die STAT1-Signalkette aktiviert, und die andere die TNFR1-Signalkette oder den CD95-Signalweg aktiviert, zur Induktion einer Seneszenz in Tumorzellen. Die Erfindung betrifft weiterhin die Behandlung und/oder Prävention von Tumoren durch Induktion eines dauerhaften Wachstumsarrests (Seneszenz). Dies erstreckt sich auch auf Tumoren die sich nicht töten lassen, d.h. bei denen sich durch eine Therapie (z.B. Chemotherapie oder Immuntherapie) keine Zytotoxizität gegenüber den Tumoren auslösen lässt.

STAT1 ("Signal Transducers and Activators of Transcription" Protein 1) ist ein Transkriptionsfaktor in humanen Zellen, der als regulatorisches Protein als Signaltransduktor direkt Signale von Rezeptoren in der Zellmembran zu den möglichen Promotoren der Zielgene im Zellkern übermitteln kann. Nach einer Stimulation mit extrazellulären Substanzen/Zytokinen bzw. nach deren Bindung an Rezeptoren, insbesondere Interferonrezeptoren, werden die STAT1-Proteine über die durch die extrazellulären Substanzen aktivierten Signalpeptid-Rezeptoren durch intrazelluläre Kinasen wiederum selber aktiviert. Dies geschieht durch eine Phosphorylierung von Tyrosinresten der STAT1-Proteine. Durch die hierdurch bewirkte Dimerisierung der STAT-Monomere werden die STAT-Dimere in den Zellkern transportiert, wo die Dimere an spezifische Promotor-Sequenzen binden und die Gentranskription initiieren können. Rezeptoren, die am STAT1-Signalweg beteiligt sind, sind z. B. die Typ I und Typ II Interferonrezeptoren (IFNR1 und/oder IFNR2), aber auch andere wie Epidermal-Growth-Factor (EGF)-Rezeptor und Platelet-Derived-Growth-Factor (PDGF)-Rezeptor; alternativ kann die Aktvierung auch über Hormone erfolgen. Daher kann durch Auswahl von Substanzen, die an diese Rezeptoren binden, der STAT1-Signalweg aktiviert werden, was erfindungsgemäß in Kombination mit der Beeinflussung des TNFR1/CD95-Signalwegs zu einer effektiven Tumorbehandlung oder -prävention führt.
Entsprechend wird vorliegend unter dem "STAT1-Signalweg" der im vorherigen Absatz beschriebene Signalweg verstanden.

Substanzen, die die STAT1-Signalkette aktivieren, sind beispielsweise Interferon-gamma (IFN-y), Interferon-alpha (IFN-α), Anti-IFNR1-Antikörper, Anti-IFNR2-Antikörper.

TNFR1 (auch als p55, CD120A benannt), ist einer der zwei Rezeptoren für Zytokine der TNF-Familie, insbesondere Tumor Nekrosefaktor (TNF-α, TNF) und Lymphotoxin (LT). TNF-α vermittelt nach Bindung an den TNFR1 in der Zelle ein breites Spektrum an Effekten wie bspw. Entzündungsreaktionen, Apoptose und Nekrose. So können über den TNFR1 - nach bisherigen Erkenntnissen - entweder Zelltod (Apoptose) oder Entzündung vermittelt werden. Eine Hemmung der TNFR1 Signalkaskade wird daher heute beispielsweise zur Behandlung entzündlicher Autoimmunkrankheiten wie Psoriasis genutzt. TNFR1 ist Teil einer Signalkettenfamilie. CD95 und TNFR1 besitzen eine weitgehend identische intrazelluläre Signalkette mit weitgehend überlappenden Signalwegen.

Dabei handelt es sich bei TNFR1, wie auch bei CD95 um ein Transmembranprotein mit einer extrazellulären Ligandenbindedomäne sowie extrazelluläre cysteinreiche Domänen, die unter anderem für die Ligandenspezifität verantwortlich sind. Bindet TNFα an TNFR1, wird dadurch eine Konformationsänderung des bis dahin inaktiven Rezeptors ausgelöst, wodurch wiederum verschiedene intrazelluläre Signalmoleküle, wie z. B. TRADD oder die Serinkinase RIP und indirekt die Tumornekrosefaktor-Rezeptor-Associated-Factors (TRAFs), an den Rezeptor binden können und dadurch aktiviert werden. Durch Bindung von TNFR1-spezifischen Liganden/Substanzen an TNFR1 kann somit dieser TNFR1-Signalweg getriggert werden. Ganz ähnliche, intrazellulär großenteils identische Signalwege können durch Bindung von CD95 aktiviert werden.

Substanzen, die die TNFR1-Signalkette oder den CD95-Signalweg aktivieren sind beispielsweise Tumornekrosefaktor-alpha (TNF-α), Anti-TNF Rezeptor1-Antikörper, Anti-CD95-Antikörper.

Eine TNFR1-Aktivierung in Kombination mit der gezielten Aktivierung des STAT1-Signalwegs durch TNF-α und IFN-γ ruft damit erfindungsgemäß eine unerwartete Wirkung sowohl in einzelnen Tumorzellen wie auch in ganzen Tumoren hervor. Die gemeinsame/koordinierte Aktivierung dieser beiden Signalwege führt zu einer neuen Signalqualität, die bislang unter keiner Bedingung beobachtet oder beschrieben wurde: Gemeinsam führt die Aktivierung der beiden Signalwege dazu, dass die Tumoren, in denen die beiden Signalwege aktiviert werden können, in einen dauerhaften Wachstumsarrest überführt werden: d.h. die Tumorzellen werden nicht getötet, sondern verlieren ihre Fähigkeit, sich wie Tumorzellen zu verhalten. In den Tumorzellen wird der Zustand des dauerhaften Wachstumsarrestes, auch Seneszenz genannt, induziert. Die Induktion dieses Wachstumsarrestes wird erfindungsgemäß zur Tumorprävention und Tumortherapie genutzt.

Wegen der weitgehend identischen intrazellulären Signalkaskaden kann auch eine Aktivierung von CD95 und STAT1 eine Seneszenz induzieren.

Entsprechend werden vorliegend unter dem "TNFR1-Signalweg" die im vorherigen Absatz beschriebenen Signalwege verstanden.

Auch wird vorliegend "Tumornekrosefaktor" mit TNF abgekürzt und abwechselnd, aber gleichbedeutend verwendet. Typ I und Typ II Interferone werden entsprechend mit "IFN" abgekürzt und gleichbedeutend verwendet.

Gegenstand der Erfindung ist daher die Verwendung einer Kombination aus zumindest zwei unterschiedlichen Substanzen, von denen die eine die STAT1-Signalkette aktiviert, und die andere die TNFR1-Signalkette oder den CD95-Signalweg aktiviert,
nämlich die Verwendung einer Kombination von Interferon-gamma (IFN-γ) mit Tumornekrosefaktor-alpha (TNF-α), zur Induktion einer Seneszenz in Tumorzellen, insbesondere in prämalignen oder malignen, bona fide Tumorzellen/Tumoren und Tumorstammzellen.

Vorliegend werden die Begriffe "Tumor" und Tumorzelle" auch synonym mit "Krebs" bzw. "Krebszelle" oder "maligne" Zelle, "Malignom" verwendet und sollen also dasselbe bezeichnen. Der Begriff "Induktion einer Seneszenz in Tumorzellen" umfasst im Sinne dieser Anmeldung sämtliche bekannten Tumorzellarten, inklusive prämalignen oder malignen, bona fide Tumorzellen/Tumoren und Tumorstammzellen.

Sowohl an TNF-Rezeptoren bindende als auch an STAT1-abhängige Rezeptoren bindende Zytokine (also bspw. TNF-α und Interferon-γ) werden als Einzelsubstanzen bereits heute zur Tumortherapie eingesetzt, wie bspw. in der EP 0 170 843 oder der EP 0 1317892 offenbart, allerdings waren die bisherigen Therapieansätze, auch jene die sich auf diesen Patentschutz beziehen, sowohl experimentell als auch therapeutisch in deren Wirkung enttäuschend bis stark begrenzt. Eine therapeutische Wirkung dieser Einzelsubstanzen, die über das Töten von Tumorzellen (Zytotoxizität) oder das Hemmen der Gefäßbildung (Anti-Angiogenese) hinausging, konnte nicht gezeigt werden; es konnten bisher keine längerfristigen therapeutischen Effekte erzielt werden.

Insbesondere war dem Stand der Technik nicht zu entnehmen, dass der Zustand der Seneszenz von Tumorzellen durch Induktion, d.h. durch die Applikation von IFN und TNF oder Substanzen die den STAT1 und TNFR1/CD95 Signalweg aktivieren, möglich ist. In vorangegangenen Patenten wie EP 0 170 843 oder der EP 0 1317892 wurde offenbart, dass durch die Kombination aus TNF und IFN Tumorzellen durch Nekrose oder Apoptose getötet werden. Mit patentierten und bisher bekannten Verfahren und Testmethoden konnte auch nur die Tötung (das Killing) von Tumorzellen erfasst werden. Da ein Töten nie alle Zellen zu 100% erfasst, regenerieren sich die Tumoren aus den überlebenden Tumorzellen und Stammzellen und wachsen anschließend wieder ungehemmt von neuem. Das Problem bilden hier die überlebenden Tumorzellen und die Tumorstammzellen aus denen sich die Tumoren wieder regenerieren (Nature 2012). Die hier vorgestellte Erfindung zeigt eindeutig, dass mit den beiden bisher zugelassenen Patenten eine effiziente Tumortherapie weder durchführbar noch planbar ist. Denn
A) IFN und TNF können in vielen Tumoren eine Zytotoxizität verursachen und diese in vitro und in vivo töten - daher der Name Tumor Necrose Faktor. Aber, das Töten führt nicht bei allen Tumoren zur Seneszenz und die bisher beschriebenen Analysemethoden (Pat I und Pat II) erlauben es auch nicht seneszente Tumorzellen zu erfassen, auch nicht per Zufall. Die Methoden erlauben auch nicht herauszufinden, ob Tumorstammzellen arretiert werden. Unsere Daten haben aber eindeutig gezeigt, dass eine Therapie nur dann langfristig wirksam ist, wenn sowohl die Tumorzellen als auch die Tumorstammzellen in einen dauerhaften Wachstumsarrest kommen.
B) Zahlreiche Tumoren lassen sich durch die Kombination aus TNF und IFN nicht abtöten. Aber sie werden durch die kombinierte Aktion von IFN und TNF in einen dauerhaften Wachstumsarrest, die Seneszenz gebracht. Die hier vorgestellte Erfindung zeigt, dass nur dieser langfristige Wachstumsarrest, der auch unabhängig vom Töten oder Killen ist, vor Tumoren schützt; dies ist vollständig unabhängig davon, ob die Tumoren durch die kombinierte Aktivierung des TNFR1 und des STAT1 Signalwegs auch gekillt werden oder nicht.

Die Erfinder der vorliegenden Erfindung haben nunmehr in eigenen Untersuchungen einen Ansatz entwickelt, der es erlaubt, in Tumoren einen therapeutisch wirksamen dauerhaften Schlafzustand bzw. Wachstumsarrest (vorliegend und im betreffenden Gebiet auch als Seneszenz bezeichnet) zu induzieren. Damit wird zum ersten Mal ein immuntherapeutischer Ansatz bereitgestellt, mittels dessen bereits maligne Tumoren in die Seneszenz, d.h. einen dauerhaften Zellzyklusarrest, überführt werden können.

Entscheidend ist dabei, dass der Therapieansatz über Signalmoleküle, die auf Zytokine antworten, in den Tumorzellen einen stabilen und dauerhaften Wachstumsstillstand, das heißt eine Tumorzellseneszenz induziert. Wichtig ist, dass der Wachstumsarrest auch noch weit über die eigentliche direkte Einwirkzeit der Induktoren/Substanzen hinausreicht. Dieses Konzept der Immuntherapie war als Therapiemaßnahme bisher nicht bekannt, bzw. wurde auch nicht angewandt.

Gegenstand der vorliegenden Erfindung ist daher auch die Verwendung einer Kombination aus zumindest zwei unterschiedlichen Substanzen, von denen die eine die STAT1-Signalkette aktiviert, und die andere die TNFR1-Signalkette oder den CD95-Signalweg aktiviert, nämlich TNF-α und IFN-γ, zur Induktion einer Seneszenz in Tumorzellen, insbesondere in prämalignen oder malignen, bona fide Tumorzellen/Tumoren und Tumorstammzellen.

Der erfindungsgemäße Einsatz der Kombination führt zur Induktion des endogenen Kinaseinhibitors p16^{lnk4a}, worüber das zellzyklusregulierende Retinoblastom (Rb) hypophosphoryliert wird, und somit eine Signalkaskade eingeleitet wird, die den Zellzyklus arretiert. Damit können auch Tumoren behandelt und in den dauerhaften Ruhezustand versetzt werden, bei welchen durch virale Genmodifikationen oder sogenannte "Tumor-Driver" Mutationen genau diese Signalkaskade gestört ist. Nur die kombinierte Aktivierung des STAT1- und des TNFR1-Signalweges bzw. die Kombination von Substanzen, die diese beiden intrazellulären Signalwege aktivieren, ist - und dies haben die Erfinder in eigenen Versuchen nachweisen können - wirksam. Im Gegensatz dazu ist, überraschenderweise, die Einzelverabreichung der Substanzen, nicht wirksam. Weder die Aktivierung von TNFR1 alleine noch die von STAT1 alleine kann einen dauerhaften Wachstumsarrest, d.h. eine Seneszenz induzieren. Sie können allenfalls das Wachstum von Tumorzellen verlangsamen - dies ist aber grundsätzlich verschieden vom Status der Seneszenz. Dies wurde eindeutig durch Experimente bewiesen. Nur die kombinatorische Verabreichung von IFN und TNF, und somit die kombinierte Aktivierung der STAT1- und TNFR1-Signalkaskade kann die Seneszenz induzieren. Entscheidend ist, dass dabei die Seneszenz auch in Tumorstammzellen induziert wird.

So konnten die Erfinder in sogenannten "RIP1-Tag2"-Mäusen, in welchen das große T-Antigen (Tag) durch Attenuation der p53- und Rb-vermittelten Zellzykluskontrolle Krebs verursacht, zeigen, dass durch die gemeinsame Aktivierung von STAT1 und TNFR1 diese Tumoren in den Ruhezustand versetzt werden, indem diese gemeinsame Aktivierung der jeweiligen Signalwege einen dauerhaften Wachstums-Stillstand in der G₁/G₀-Phase induziert. Sie zeigen, dass die gemeinsame Aktivierung von STAT1 und TNFR1, und nur die gemeinsame Aktivierung von STAT1 und TNFR1, zur Erhöhung des p16^{lnk4a} Proteins führt, wodurch Rb im hypophosphorylierten Zustand vorliegt und dadurch anschließend E2F2-Gene unterdrückt werden.

Es ist bisher nicht bekannt oder beschrieben, dass Botenstoffe wie Zytokine, bspw. Interferone oder TNF, Antikörper oder andere exogene oder endogene Substanzen einen dauerhaften Wachstumsarrest in Tumorzellen auslösen können. Letzteres ist nur für einige Chemotherapeutika bekannt, deren Verabreichung mit starken Nebenwirkungen einhergeht.

Die Erfinder konnten aber zusätzlich noch drei weitere entscheidende Aspekte zeigen, die die Neuartigkeit des Ansatzes unterstreichen: Die Induktion der Seneszenz über die Aktivierung von STAT1 und TNFR1 in Tumoren *in vivo* führt dazu, dass die Tumorzellen nicht mehr wachsen können, auch wenn sie aus den behandelten Tieren isoliert werden und somit keine direkte Behandlung mehr stattfindet. Selbst wenn sie danach in schwer immundefiziente Mäuse transplantiert werden, wachsen sie nicht mehr. Sie verhalten sich wie echt benigne Zellen (trotz des enthaltenen Tumorgens); auch Tumorstammzellen können durch die Seneszenzinduktion dauerhaft stillgestellt werden. Ferner konnten die Erfinder durch genetische Analysen mittels chromosomaler Gen-Hybridisierung zeigen, dass die Seneszenzinduktion durch STAT1 und TNFR1 das Genom der Tumorzellen und ihrer potentiellen Vorläufer stabilisiert. Außerdem wurde nachgewiesen, dass die Aktivierung von STAT1 und TNFR1 auch bei anderen Tumoren der Maus, die als Modell für Tumoren des Menschen dienen, eine Seneszenz induzieren; ferner wurde an 6 unterschiedlichen Tumorzelllinien und mehreren frisch isolierten Tumoren des Menschen gezeigt, dass dies auch für Tumoren des Menschen gilt.

Der Zellzyklus wird üblicher Weise in vier Abschnitte untergliedert, nämlich in die G₁-Phase, die S-Phase, die G₂-Phase und in die M-Phase. In der G₁-Phase findet die Vorbereitung für die DNA-Replikation statt, in der sich daran anschließenden S-Phase werden hauptsächlich die DNA dupliziert und andere wichtige Zellbausteine wie z. B. Phospholipide synthetisiert, in der sich anschließenden G₂-Phase wird die Integrität des Genoms kontrolliert und die Zellteilung vorbereitet, und in der abschließenden mitotischen (M) Phase wird das duplizierte Genom gleichmäßig auf die Tochterzellen verteilt. Vor Beginn der nächsten G₁-Phase können die Zellen den geschilderten Zellzyklus verlassen und in die sog. Ruhephase (G₀) eintreten, in der sie durch Differenzierung aus dem Zellzyklus entfernt werden. Die G₀-Phase ist reversibel, und die Zellen können durch bestimmte, Mitose auslösende Signale, wie z. B. Wachstumsfaktoren, Tumorviren, wieder in die G₁-Phase zurückkehren und dann am Zellzyklus teilnehmen.

In der G₀- und G₂-Phase sind DNA-Reparaturen möglich, die die genetische Integrität der Zelle sicherstellen und diese vor einer unkontrollierten Proliferation schützen. Proteine wie Rb (Retinoblastom) und p53 ermöglichen den Übergang in diese Ruhephase. Störungen und Mutationen im Bereich der p53 oder Rb Signalkaskade führen zur unkontrollierten Zellproliferation und darüber hinaus langfristig zu malignen Tumoren. An der Kontrolle des Zellzyklusses sind ferner u.a. Zyklin-abhängige Kinasen (CDK 1 - 8) und ihre Inhibitoren (CDKIs) beteiligt, indem sie Komplexe mit jeweils spezifischen Zyklinen bilden.

Im Zellzyklus inhibitorisch wirken u.a. zwei unterschiedliche Proteinfamilien: Mitglieder der p16^{lNK4}-Familie hemmen CyklinD/CDK4/CDK6-Komplexe in der frühen S-Phase, und Moleküle der P21cip/waf-Familie wirken in der späten G1-Phase. Sie fungieren als Gegenregulatoren des oben genannten Systems und bremsen die zelluläre Proliferation. Fallen diese negativen Regulatoren bspw. durch Mutation oder Deletion der Gene oder durch eine gestörte Genexpression aus, wird eine maligne Entartung von Zellen hervorgerufen.

Die Regulation des Zellzyklus erfolgt, wie oben erwähnt, durch die Zyklin-abhängigen Kinasen (Cdks) und verschiedene Zykline, die zu definierten Zeiten im Zellzyklus mit verschiedenen Kinaseuntereinheiten assoziieren, wodurch ein Netzwerk aus Kinaseaktivitäten entsteht, das durch kontrollierte Phosphorylierung von Zielproteinen den Verlauf des Zellzyklus reguliert. U.a. wird durch die Phosphorylierung des Retinoblastom-Proteins (Rb-Protein), das unphosphoryliert den Transkriptionsfaktor E2F2 blockiert, E2F2 freigesetzt, wodurch dieser Transkriptionsfaktor Gene des Zellzyklusses aktivieren kann.

Mittels des Seneszenzmarkers "Phosphorylated Heterochromatin Protein 1γ" pHP1γ oder der Seneszenz-assoziierten β-Galactosidase (SA-β-gal) wurde nachgewiesen, dass in der Tat durch die gleichzeitige Aktivierung von STAT1 und TNFR1 ein stabiler Wachstumsstillstand, also der Eintritt der Tumorzellen in die G₀-Phase, induziert werden konnte. Dies wurde durch Zellzyklusanalysen bestätigt; nur nach der kombinierten Aktivierung der STAT1- und TNFR1-Signalwege verließen die Tumorzellen die S-Phase und reicherten sich in der G₀-Phase an.

Weiter konnte durch kombinatorischen Einsatz zweier Substanzen, die den STAT1- bzw. den TNFR1-Signalweg aktivieren, ein permanenter Wachstumsarrest von β-Krebszellen erreicht werden, wohingegen die Aktivierung entweder der einen oder der anderen Signalkaskade zwar das Zellwachstum verlangsamen kann, auf keinen Fall aber einen vollständigen und insbesondere keinen dauerhaften, über die Anwendung der Zytokine hinaus anhaltenden Wachstumsarrest induzieren kann. Experimentell konnte gezeigt werden, dass der *in vivo* induzierte, dauerhafte Wachstumsarrest (die Seneszenz) über mehrere Monate stabil blieb - so lange wie das Experiment zeitlich-technisch durchführbar war.

In weiteren Ausführungsformen kann vorgesehen sein, die Substanzen mittels Immunzellen, DNA/RNA Small Particles, Nanopartikel, Virotherapie oder Immuntherapie direkt an die Tumoren heranzubringen oder in die Tumorzellen einzubringen und dadurch ihre effektive Dosis am Wirkort zu erhöhen.

Erfindungsgemäß erfolgt also keine Tumor-Tötung (z.B. Apoptose, Lyse, Nekrose) oder Antiangiogenese, sondern ein gezielter Wachstumsarrest. In einer bevorzugten Ausführungsform besteht die Kombination aus TNF(Tumor-Nekrose-Faktor)-α und Interferon-γ.

Erfindungsgemäß werden alternative Konzentrationen der Substanzen bzw. der Kombination eingesetzt, durch welche kein Zelltod erreicht werden soll (er kann bei dieser Kombination eintreten, ist aber nicht das Ziel der Therapie und dafür auch nicht nötig), sondern ein dauerhafter Zellzyklusarrest bzw. die Seneszenzinduktion. Dadurch werden die Tumoren in ihrem Genom stabilisiert, d.h. gemäß der Erfindung wird die genomische Entartung der Tumorzellen und ihrer Vorläufer behindert. Erfindungsgemäß wird also erreicht, dass die Tumorzellen ihre normale funktionale Differenzierung beibehalten bzw. wieder erlangen. Dies war nicht das Ziel der bisherigen, auf die Tötung der Tumorzellen gerichteten Therapien. Durch herkömmliche Therapien wurde sogar oft erreicht, dass die Tumorzellen phänotypisch weiter entarten. Die herkömmlichen Immuntherapieansätze erlauben auch weder das Ziel der Seneszenz anzustreben noch dieses Ziel duch Messung zufällig zu entdecken.

Ein weiterer Gegenstand der Erfindung ist daher die Verwendung einer Substanzkombination, von denen eine die STAT1-Signalkette aktiviert und die andere die TNFR1-Signalkette oder die CD95-Signalweg aktiviert, nämlich TNF-α und IFN-γ, zur Verhinderung der weiteren Entartung von Tumorzellen. Ein weiterer Aspekt der Erfindung ist die Verwendung einer Substanzkombination, von denen eine die STAT1-Signalkette aktiviert und die andere die TNFR1-Signalkette oder den CD95-Signalweg aktiviert, nämlich TNF-α und IFN-γ, zur Wiedererlangung der funktionalen Differenzierung einer Tumorzelle.

Erfindungsgemäß wird die Kombination bei Tumoren eingesetzt, bei welchen die STAT1- und TNFR1- Signalkaskade aktivierbar ist und p16^{lnk4a} präsent ist. Erfindungsgemäß nicht behandelbar sind Tumore mit einer nicht kompensierten Deletion des p21-p19-p16/p14AARF Komplexes.

Der zu behandelnde Tumor ist dabei vorzugsweise ein Tumor, der durch die kombinierte Aktivierung der STAT1-Signalkette und der TNFR1-Signalkette in einen dauerhaften Ruhezustand versetzt werden kann, und ist vorzugsweise ausgewählt aus Tumoren wie HPV-induzierte benigne Tumoren, Präkanzerosen und Karzinome, Brustkrebs, Sarkome, Melanome, Karzinome (Ovarien, Zervix, Haut- und Schleimhäute, Prostata, Niere, Lunge), ZNS-Tumoren oder Lymphome/Leukämien. Erfindungsgemäß ist die effiziente Aktivierung der TNFR1- und der STAT1-Signalkaskade möglich und p16^{lnk4a} funktionell erhalten; andere häufige Störungen der Seneszenz-Signal-Kaskade, wie eine Inhibition von p53 oder Rb, beispielsweise durch Tag wie es in HPV-induzierten Tumoren vorkommt, können gerade durch die effiziente Aktivierung von STAT1 und TNFR1 kompensiert werden; dies wurde von den Erfindern experimentell belegt.

Wie von den Erfindern experimentell dargelegt kann eine Tumorzelle durch Kontakt mit der erfindungsgemäßen Substanzkombination in einer Konzentration von 0,0001 ng/ml bis 10.000 ng/ml in den Zustand der Seneszenz überführt werden. Sobald die Seneszenz eingetreten ist verharrt die Zelle in diesem Zustand, auch wenn die erfindungsgemäßen Substanzen nicht weiter appliziert werden. Dies gilt insbesondere auch für Tumorzellen, in denen sich durch die kombinierte Aktion von IFN und TNF/CD95 keine Zytotoxizität oder - Apoptose auslösen läßt, und die durch die kombinierte Aktivierung der TNFR1/CD95 und STAT1 Signalkaskade nicht getötet werden können.

Für Zwecke der in-vivo Behandlung ist daher sicherzustellen, dass alle Tumorzellen gemäß der Lehre der vorliegenden Erfindung in den Zustand der Seneszenz überführt werden. Die Behandlung muss daher sicherstellen, dass die erfindungsgemäßen Substanzen im oben genannten Konstellationsbereich am Wirkort (d. h. an jeder einzelnen Tumorzelle) erreicht werden. Dies wird dadurch sichergestellt, dass die Behandlung durch die erfindungsgemäße Substanzkombination mit einer Konstellation von 0,0001 ng/ml bis 10.000 ng/ml über mindestens 3-4 Tage andauert. Sollte ein Patient bereits Tumore mit einem Durchmesser von 5 mm oder größer entwickelt haben, dann sollte die Behandlung bis zu 30 Tage andauern. Um zu verhindern, dass möglicherweise einzelne Zellen nicht in den Zustand der Seneszenz überführt wurden (beispielsweise weil keine ausreichende Konzentration der erfindungsgemäßen Substanzkombination am Wirkort erreicht wurde) kann die initiale Behandlung im Abstand von 3 - 6 Wochen wiederholt werden. Dabei empfiehlt sich eine engmaschige Beobachtung ggf. bereits gebildeter Tumore. Sollten diese nach der Behandlung mit der erfindungsgemäßen Wirkstoffkombination weiter wachsen, kann die erfindungsgemäße Behandlung auch durch peritumorale oder intratumorale Applikation erfolgen. Auf diese Weise kann sichergestellt werden, dass die erfindungsgemäß notwendige Wirkstoffkonzentration am Wirkort, nämlich an der einzelnen Tumorzelle, erreicht wird.

Die vorliegende Erfindung betrifft daher auch eine pharmazeutische Zusammensetzung, die eine Kombination aus Substanzen enthält, von denen die eine die STAT1-Signalkette aktiviert, und die andere die CD95/TNFR1-Signalkette aktiviert, nämlich TNF-α und IFN-γ, zur Behandlung und/oder Prävention von Tumoren, wobei die Substanzen in der pharmazeutischen Zusammensetzung als isolierte Einzelsubstanzen vorliegen können.

Ein besonders bevorzugter Gegenstand der Erfindung ist eine pharmazeutische Zusammensetzung enthaltend eine Kombination von Interferon-gamma (IFN-γ) mit Tumornekrosefaktor-alpha (TNF-α).

Ferner ist bevorzugt, wenn gemäß einer Ausführungsform der Erfindung die Substanzen jeweils in einer Konzentration von 0,0001 ng/ml bis 10.000 ng/ml in der pharmazeutischen Zusammensetzung bzw. auch am Wirkort enthalten sind.

Neben der erfindungsgemäßen Kombination können weitere Hilfsmittel oder Zusätze, additive oder Träger enthalten sein, die die jeweilige Verabreichung erleichtern, bzw. vereinfachen oder möglich machen.

Die im Rahmen der Erfindung einzusetzende therapeutisch wirksame bzw. therapeutisch effektive Menge der kombinatorisch einzusetzenden Substanzen ist dabei diejenige Menge der Kombination aus zumindest zwei Substanzen, wie obenstehend erläutert, welche die Seneszenz, d.h. also den permanenten Ruhezustand, des Tumors induzieren kann. Die genaue effektive Menge für einen Patienten hängt ab von dessen Größe und Gesundheitszustand, der Lokalisation, der Art und dem Ausmaß der Tumorerkrankung sowie der Kombination der einzusetzenden Substanzen in der pharmazeutischen Zusammensetzung.

Die pharmazeutische Zusammensetzung kann einem Patienten in einer Vielzahl von Formen verabreicht werden, die dem gewählten Weg der Verabreichung, nämlich parenteral, oral, intraperitoneal, transdermal etc. angepasst sind. Eine parenterale Verabreichung schließt dabei die Verabreichung auf folgenden Wegen ein: intravenös, intramuskulär, interstitiell, intraarteriell, subkutan, intrasynovial, transepithelial, einschließlich transdermal, pulmonal, olphtalmisch sublingual und bucal, topisch einschließlich dermal, okular, rektal sowie eine nasale Inhalation via Insufflation oder peri-/intratumoral ein.

Die Verabreichung kann dabei in Form von Lösungen, Tinkturen, Salben, Pulvern und Suspensionen, oder weiteren festen oder flüssigen Formulierungen sowie als Tabletten, Kapseln, Spray, Viren, verpackte, gebundene oder freie DNA oder RNA, Nanopartikel, oder Zellen wie T- oder NK-Zellen verabreicht werden.

Wie ferner weiter oben erwähnt, kann die pharmazeutische Zusammensetzung auch pharmazeutisch akzeptierbare Träger, Verbindungsmittel, Verdünnungsmittel, Exzipienten oder Adjuvantien aufweisen. Die Wahl eines pharmazeutischen Trägers, Exzipienten oder anderen Hilfsmittels kann im Hinblick auf den beabsichtigten Verabreichungsweg und die standardisierte pharmazeutische Praxis vorgenommen werden. Als pharmazeutisch akzeptierbare Träger können Lösungsmittel, Streckmittel oder andere flüssige Bindemittel wie Dispersions- oder Suspensionshilfsmittel, oberflächenaktive Agentien, isotonische Wirkstoffe, Deckungsmittel oder Emulgatoren, Konservierungsmittel, Umhüllungsmittel, feste Bindestoffe oder Gleitmittel eingesetzt werden, je nachdem, was für die jeweilige Dosierung am besten geeignet ist und zugleich mit der erfindungsgemäßen Kombination kompatibel ist. Eine Übersicht über solche zusätzliche Inhaltsstoffe findet sich bspw. in A. Kibbe: Handbook of Pharmaceutical Excipients, 3rd Edition, 2000, American Pharmaceutical Association and Pharmaceutical Press.

Die erfindungsgemäße pharmazeutische Zusammensetzung kann auch pharmazeutisch akzeptierbare Salze aufweisen, wie bspw. Salze von Mineralsäuren, wie Hydrochloride, Hydrobromide, Phosphate, Sulfate und vergleichbare; aber auch Salze organischer Säuren.

Wie weiter oben erwähnt, erfolgt eine bevorzugte therapeutische Verabreichung der pharmazeutischen Zusammensetzung, im Falle von Zytokinen, im Bereich von 0,0001 ng/ml bis 10.000 ng/ml in der pharmazeutischen Zusammensetzung am Wirkort, wobei eine therapeutisch effektive tägliche Dosis der erfindungsgemäßen Kombination im Bereich des vorhandenen oder sich entwickelnden Tumors erreicht werden muss, vorzugsweise 0,001 µg bis 1000 mg/kg Tumorgewebe. Werden immunaktivierende Antikörper, oder immunstimulatorische Moleküle (wie immunstimulatorische DNA Motive; exogene oder endogene Aktivierung der "Innate Immunity") verwendet, richtet sich die Dosierung an der Stärke aus mit der eine effiziente T oder NK Zellaktivierung erfolgt. Typischerweise wird der Arzt die tatsächliche Dosis bestimmen, die für einen bestimmten Patienten benötigt wird, was in der Abhängigkeit von der Applikationsart, seines Alters, des Gewichts und dem allgemeinen Gesundheitszustand des Patienten geschehen wird. Dabei wird er die mögliche endogene Produktion der Wirkstoffe, beispielsweise von TNF-α berücksichtigen: In Tumoren in denen das TNF-α bereits in ausreichender Menge endogen gebildet wird, kann gegebenenfalls auch auf die Zugabe von weiterem TNF-α verzichtet werden.

Im Rahmen der vorliegenden Anmeldung wurde erstmalig die pharmakologische Induktion einer Seneszenz von Tumorzellen beschrieben. Eine derartige Induktion der Seneszenz bei Tumorzellen war bislang nicht beschrieben. Die Seneszenzinduktion erstreckt sich insbesondere auch auf Tumoren, die sich durch die Wirkstoffkombination nicht töten lässt und auf Tumorstammzellen, die gegenüber fast allen anderen Therapieverfahren resistent scheinen. Die im Rahmen der vorliegenden Erfindung geschilderten Ergebnisse ermöglichen daher auch den Aufbau eines Screening-Assays zur Untersuchung pharmazeutischer Substanzen auf ihre Eignung zur Induktion einer derartigen Seneszenz. Dabei können Einzelsubstanzen, bevorzugt aber Substanzkombinationen mit Tumorzellen in Kontakt gebracht werden und die Tumorzellen anschließend untersucht werden, ob das Stadium der Seneszenz erreicht wurde. Bejahendenfalls können die Einzelsubstanzen bzw. Substanzkombinationen daraufhin untersucht werden, ob sie als pharmazeutische Wirkstoffe geeignet sind. Gegenstand der vorliegenden Erfindung ist daher auch ein Verfahren zum Screenen von Wirkstoffen und Wirkstoffkombinationen auf die Induktion einer Seneszenz in Tumorzellen.

Ein weiterer Aspekt der vorliegenden Erfindung ist die Möglichkeit den speziellen Tumortyp eines speziellen Patienten vor der Behandlung daraufhin zu untersuchen, ob und ggf. welcher Wirkstoff bzw, welche Wirkstoffkombination dafür geeignet ist, in diesem speziellen Tumortyp eine Seneszenz zu induzieren. Hierzu kann eine Gewebeprobe des Patienten (beispielsweise aus einer Biopsie) dem oben geschilderten Verfahren unterzogen werden. Dies bedeutet, dass isolierte, einzelne Tumorzellen einer Wirkstoffkombination ausgesetzt werden und anschließend daraufhin untersucht werden, ob eine Seneszenz induziert wurde.

Beschrieben ist daher auch ein Verfahren zum Testen von Tumoren auf die Möglichkeit einer Behandlung durch pharmakologisch induzierte Seneszenz, dadurch gekennzeichnet, dass Tumorzellen durch Biopsie entnommen werden, die isolierten Tumorzellen über einen Zeitraum von 4 bis 14 mit einer Lösung, enthaltend die zu untersuchenden Wirkstoffe in einer Konzentration von 0,0001 ng/ml bis 10.000 ng/ml versetzt werden, und anschließend daraufhin untersucht werden, ob eine Seneszenz eingetreten ist.

Weitere Vorteile ergeben sich aus der nachstehenden Beschreibung sowie den beigefügten Figuren.

### Beispiele

Die Erfindung wird durch die nachfolgend geschilderten Beispiele weiter erläutert.

Einige Ausführungsbeispiele der Erfindung sind in den Figuren dargestellt und werden in der nachfolgenden Beschreibung näher erläutert. Diese zeigen:

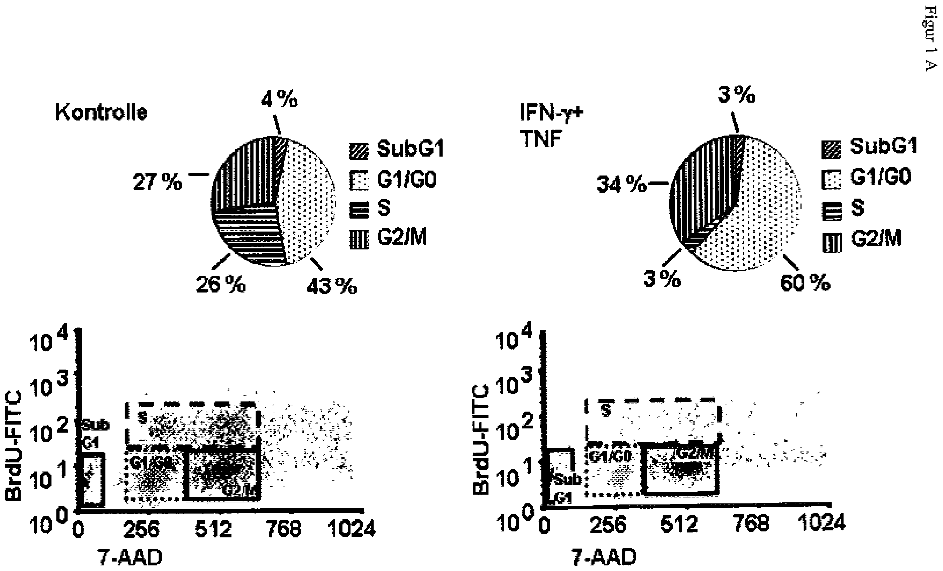

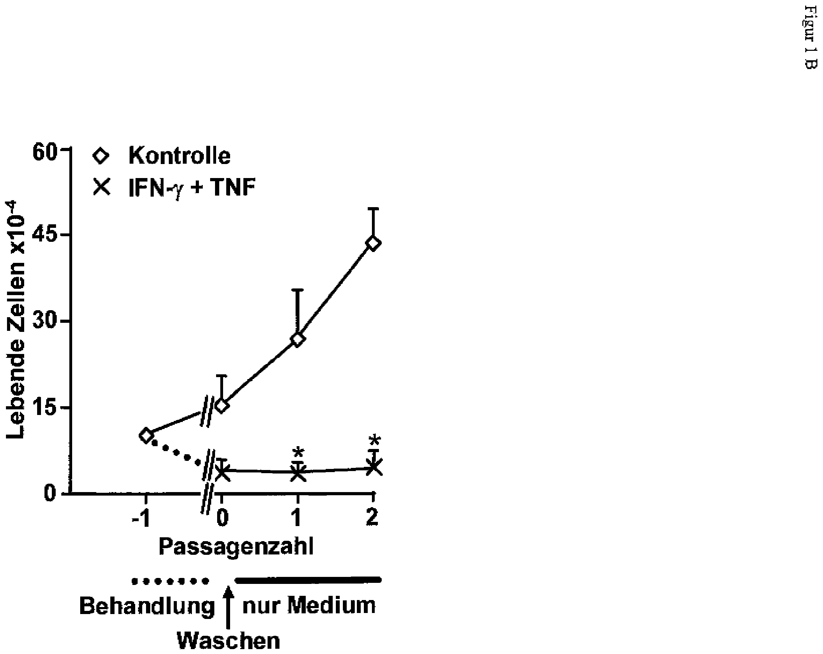

- Fig. 1: einen FACS-Plot, und ein Diagramm, welchen zu entnehmen ist, dass die Kombination aus zwei Substanzen, welche die STAT1- und die TNFR1-Signalkaskade aktivieren (hier: Interferon-γ und TNF) Krebszellen in der G₁/G₀-Phase arretierten, ohne diese zu töten - es findet sich keine Anreicherung der Zellen in der sub-G1 Phase (rechte Abbildung, **(A)**). Zellzyklusmessungen an unbehandelten und mit einer erfindungsgemäßen Kombination (STAT1- und TNFR1-Aktivatoren) behandelten β-Krebszellen **(B).** Die β-Krebszellen wurden nicht nur arretiert (A rechter und B linker Teil der Graphik), sondern auch nach Entfernung der Seneszenz-induzierenden Signale definitionsgemäß weiter in einem dauerhaften Wachstumsstillstand gehalten **(B);**

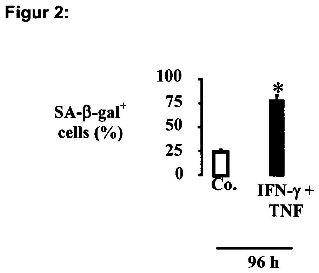

- Fig. 2: den Nachweis, dass eine erfindungsgemäße Kombination von STAT1- und TNFR1-Aktivatoren (hier: Interferon-γ und TNF) die Seneszenzassoziierte β-Galactosidase (SA-β-gal)-Aktivität in den Tumorzellen veränderten: Das Diagramm zeigt, dass die kombinierte Verabreichung von Interferon-γ und TNF die SA-β-gal-Aktivität in β-Krebszellen stark induziert;

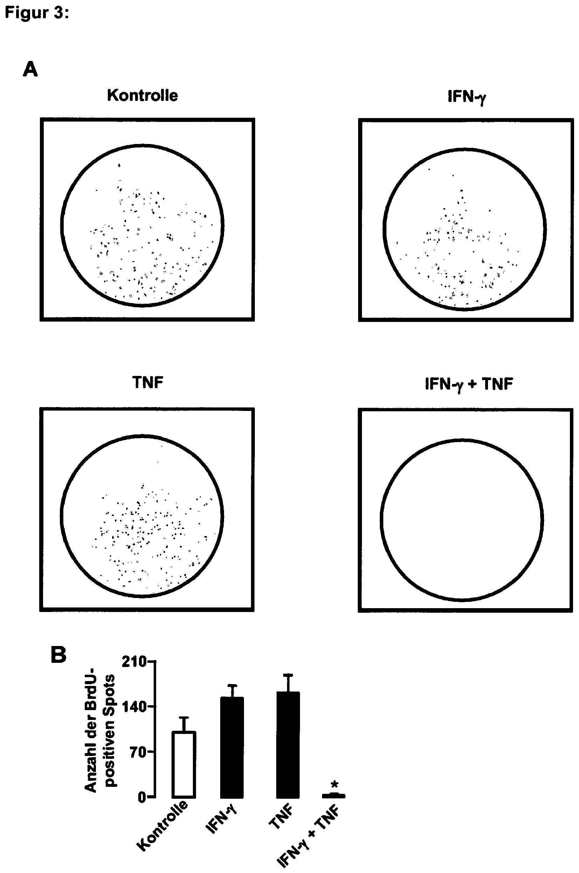

- Fig. 3: den Nachweis, dass nur eine kombinierte Aktivierung von STAT1 und TNFR1 einen stabilen Wachstumsarrest in isolierten β-Krebszellen *in vitro* induziert, jedoch nicht eine Aktivierung jeweils nur eines der beiden Signalwege: **A** zeigt, dass bei einer Behandlung von β-Krebszellen, die 4 bis 5 Tage entweder mit Medium (Co.), Interferon-γ, TNF, oder einer Kombination aus Interferon-γ und TNF kultiviert wurden, nur β-Zellen, die mit der Kombination behandelt wurden, in den permanenten Ruhezustand versetzt wurden; **B** zeigt die Anzahl an BrdU-positiven β-Krebszellen nach der Behandlung mit Medium (Co.), Interferon-γ, TNF, oder der Kombination aus Interferon-γ und TNF, wobei zum Zeitpunkt der Messung die Zytokine dem Medium bereits seit 2 Wochen entzogen wurden.

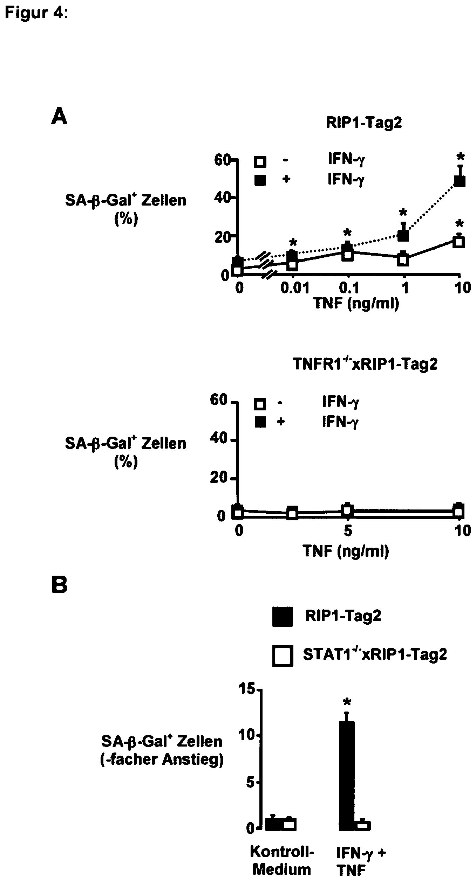

- Fig. 4: den Nachweis, dass für die Seneszenzinduktion durch die Kombination aus IFN-γ und TNF unbedingt gemeinsam die STAT1- und TNFR1-Signalkaskade aktiviert werden müssen. In TNFR1- (Fig. 4**a**) oder STAT1- (Fig. 4**b**) defizienten β-Krebszellen kann die Seneszenz durch Interferon-γ und TNF nicht induziert werden;

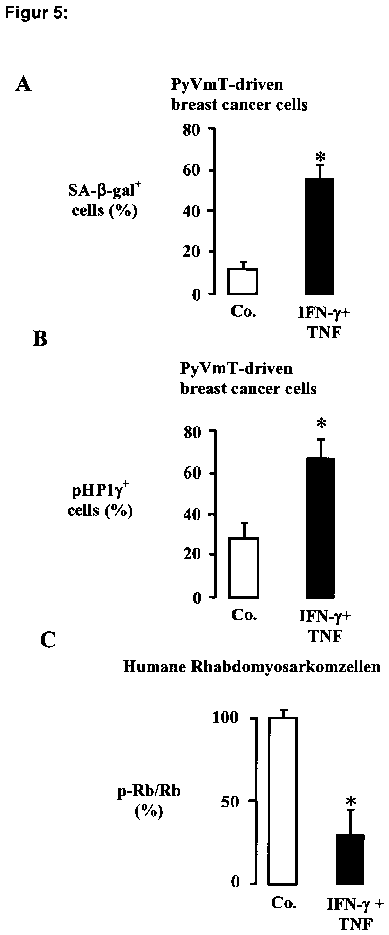

- Fig. 5: den Nachweis, dass eine kombinierte Verabreichung zweier Substanzen (hier: Interferon-γ und TNF) sowohl in Brustkrebszellen (Fig. 5**a** und **b**) als auch bei Rhabdomyosarkomzellen des Menschen die Seneszenz induziert (siehe Fig. 5**c**).

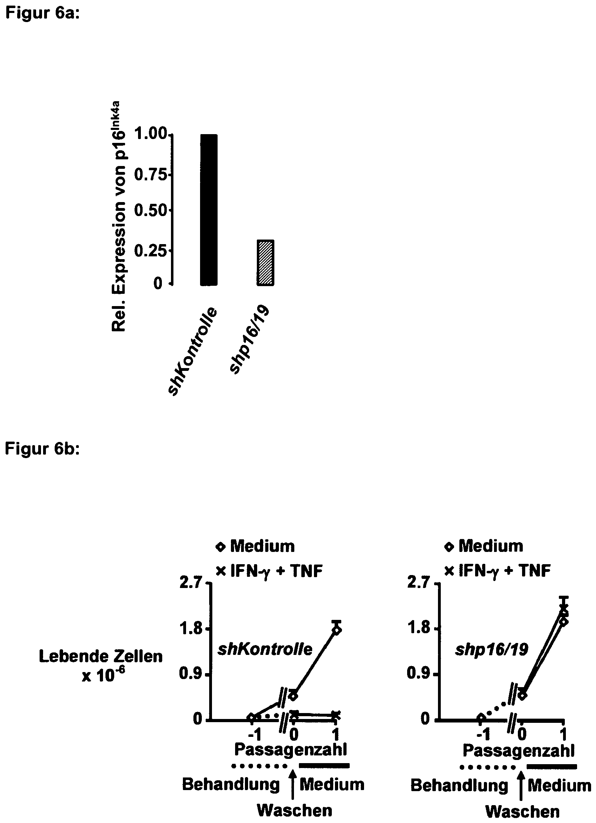

- Fig. 6: den Nachweis, dass die Seneszenzinduktion strikt abhängig ist von der Gegenwart des p16^{lnk4a} Proteins; elektiv in Tumorzellen, in denen p16 durch shRNA blockiert wurde (siehe Fig. 6a), kann die Kombination aus Interferon-γ und TNF keine Seneszenz induzieren (Fig. 6b: rechtes Bild shp16 x-Symbol); dies steht im Gegensatz zur Induktion von Lyse, Apoptose oder Nekrose von Tumorzellen durch IFN-γ und TNF;

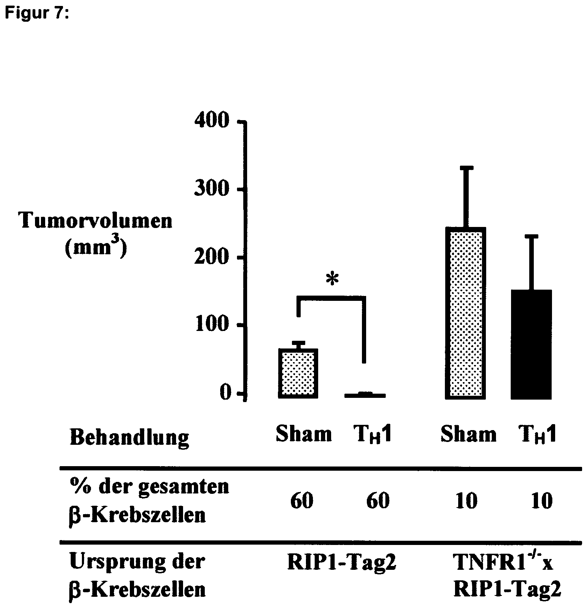

- Fig.7: den Nachweis, dass die *in vivo*-induzierte Seneszenz über Monate anhält und von der Gegenwart des TNFR1 auf den Tumorzellen abhängig ist; TNFR1-abhängig führt die Immuntherapie *in vivo* dazu, dass die Tumorzellen immer arretiert sind, auch wenn sie in immunkompromittierte Mäuse überführt werden; die seneszenten Zellen verhielten sich trotz Expression des Tumor-Driver Gens somit wie benigne Zellen - und nicht mehr wie Tumorzellen; dies illustriert insbesondere auch erstmals, dass hierdurch die Tumorstammzellen in einen dauerhaften Wachstumsarrest überführt werden konnten;

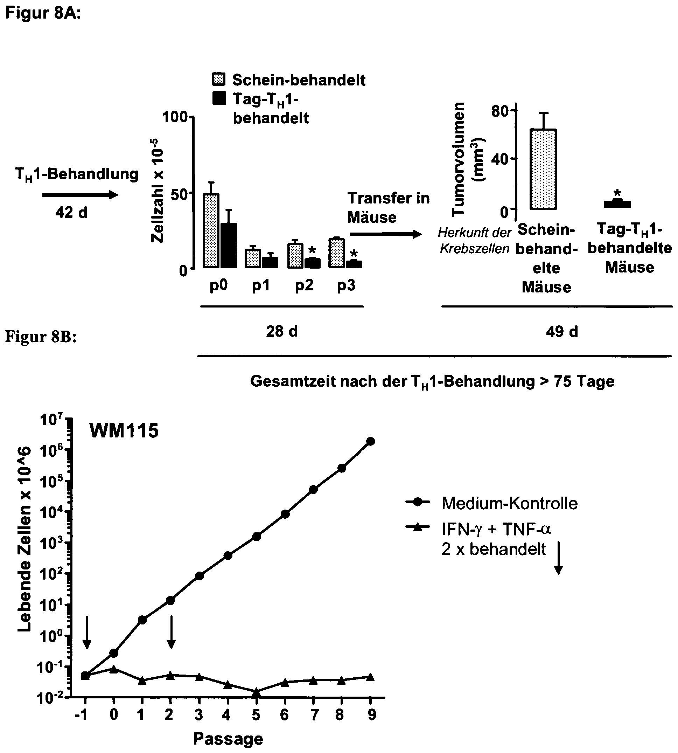

- Fig. 8A: den Nachweis, dass eine Transplantation von arretierten Tumorzellen in immundefiziente Mäuse den Tumorarrest nicht aufhebt und somit kein Tumorwachstum mehr zu beobachten ist (>75 Tage);
- Fig. 8B: den Nachweis des Tumorarrests nach Behandlung von WM115 Melanomzellen mit IFN-γ und TNF über 10 Passagen (>45 Tage);

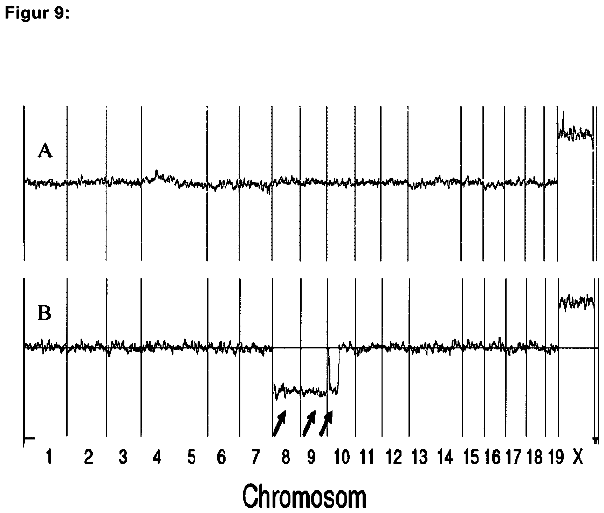

- Fig. 9: den Nachweis, dass die kombinierte Aktivierung von STAT1 und TNFR1 das Genom der Tumorzellen stabilisiert: Das obere der beiden Beispiele zeigt Tumoren aus einem behandelten Tier ohne GenomAberrationen (in der CGH-Analyse), und das untere der beiden Beispiele zeigt Tumoren aus einem gleichaltrigen scheinbehandelten Kontrolltier mit Genom-Aberrationen;

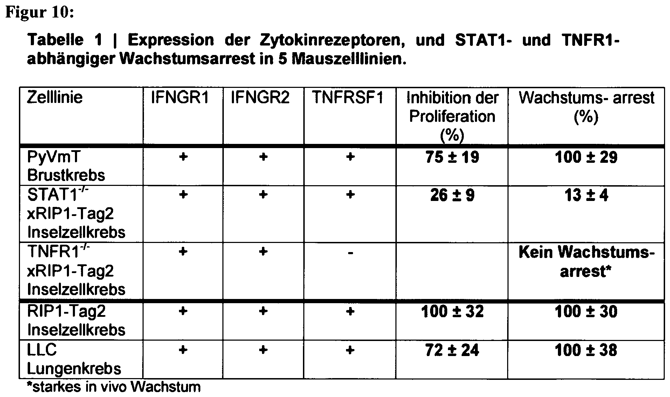

- Fig. 10: in Tabelle 1 den Nachweis an 3 Tumoren der Maus, dass schwere Störungen in der p53-Rb Seneszenzkaskade (wie deren Inhibition in RIP-TAG oder PyVmt Tumoren) über die Seneszenzinduktion kompensiert werden konnten; weiterhin ist gezeigt, dass die gemeinsame Aktivierung von STAT1 und TNFR1 nötig war, um die Seneszenz zu induzieren, denn das Fehlen einer der beiden Signalwege hat die Seneszenz Induktion *in vivo* und *in vitro* wieder aufgehoben.

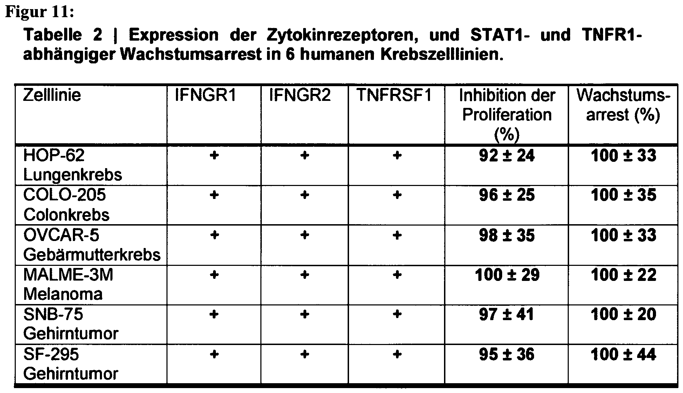

- Fig. 11: den Nachweis in 6 verschiedenen Tumorzell-Linien, dass die kombinierte Aktivierung von STAT1 undTNFR1 in verschiedenen Tumoren des Menschen eine Seneszenz induzierte.

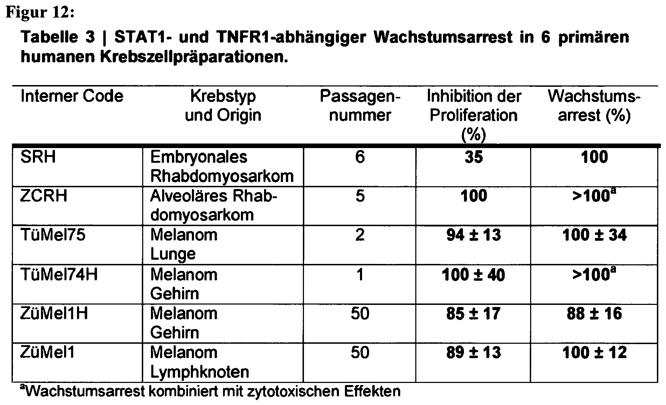

- Fig. 12: den Nachweis in 4 verschiedenen frisch isolierten Tumoren des Menschen, dass die kombinierte Aktivierung von STAT1 und TNFR1 in verschiedenen Tumoren des Menschen eine Seneszenz induzierte.

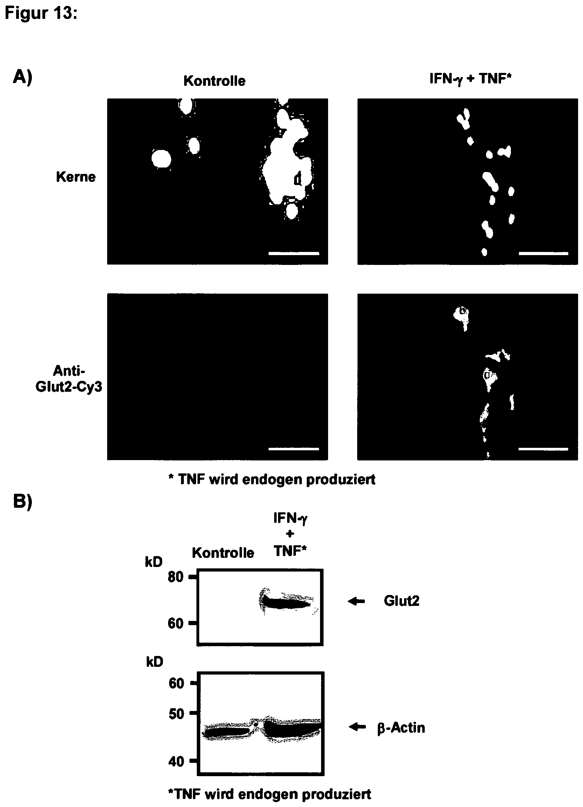

- Fig. 13: den Nachweis, dass isolierte, gänzlich entdifferenzierte β-Krebs-Zellen in Anwesenheit der T_{H}1-Zytokine IFN-γ und TNF wieder zu bona fide Inselzellen redifferenzieren

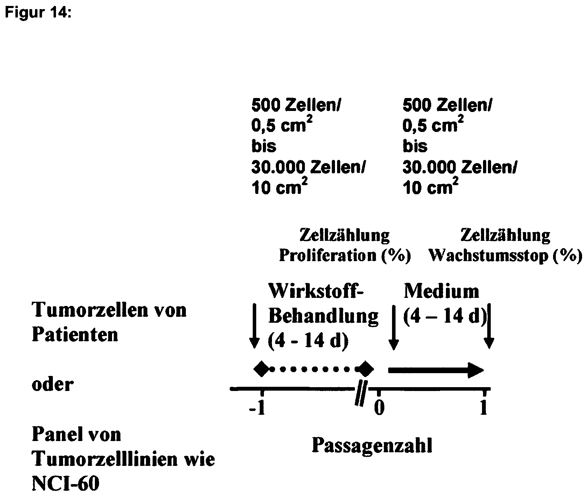

- Fig. 14: ein Ausführungsbeispiel zum Nachweis der eingetretenen Seneszenzinduktion

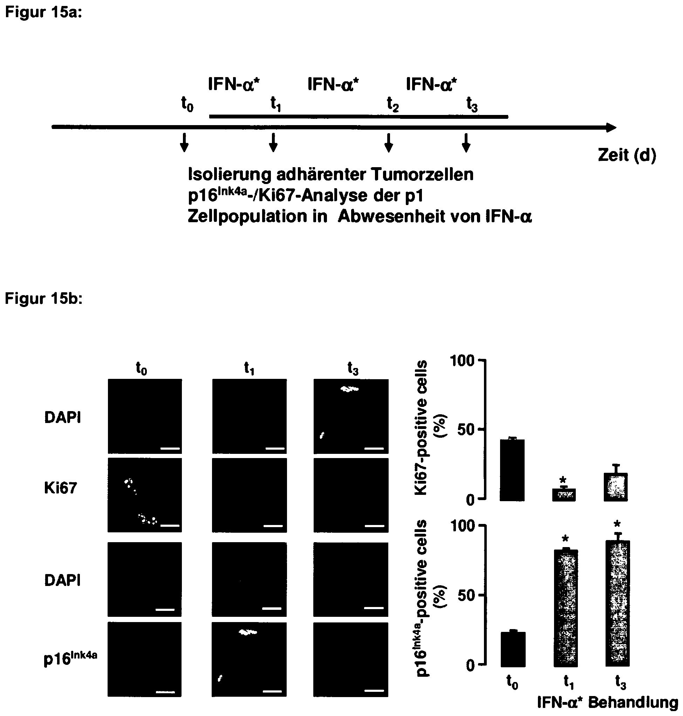

- Fig 15: das Ergebnis eines Heilversuchs an einem Humanpatienten mit Peritonealkarzinom mit IFN-α und TNF: Es kommt zu einer starken Induktion von p16^{lnk4a} nach der Behandlung sowie einer deutlichen Abnahme des Proliferationsmarkers Ki67. Zum Nachweis der Zellen wurden die Zellkerne zusätzlich mit DAPI gefärbt.

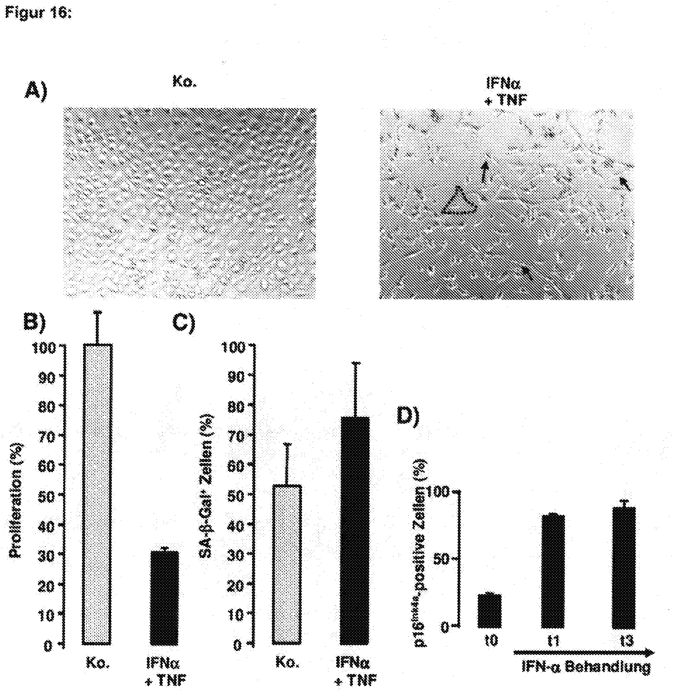

- Fig.16: weitere Ergebnisse des Heilversuchs: Die IFN-α und TNF behandelten Tumorzellen ändern sich in Anzahl und Morphologie (16A), zeigen eine verminderte Proliferationsrate (16B,) zeigen eine sehr hohe Aktivität der Seneneszenz-assoziierten β-Galaktosidase (SA-β-Gal, 16C) sowie eine starke Expression von p1^{lnk4a} (16D).

### Methoden

Tiere: Für die Versuche wurden C3H-Mäuse (Charles River, Sulzberg, Deutschland), transgene RIP1-Tag2-Mäuse, doppeltransgene TNFR1^{-/-} und STAT1^{-/-}Mäuse eingesetzt.

Zellkultur und Einzelzellanalyse: Tag-spezifische T_{H}1-Zellen wurden isoliert und mittels Durchflusszytometrie charakterisiert. Tumoren wurden aus Sham- und mit Tag-T_{H}1-Zellen behandelten RIP1-Tag2-Mäusen isoliert, sowie aus Sham- und mit Tag- T_{H}1-Zellen behandelten TNFR1^{-/-}xRIP1-Tag2-Mäusen, STAT1^{-/-}xRIP1-Tag2-Mäusen oder aus transgenen Brustkrebsmäusen mittels Collagenaseverdau (10 min bei 37 °C) isoliert, und anschließend unter einem Dissektionsmikroskop getrennt. Die Tumorzellen wurden durch Inkubation in 0,05 % Trypsin/EDTA-Lösung (10 min bei 37 °C) gewonnen und auf Gewebekulturplatten ausgesät. Adhärente Zellen wurden 2 bis 5 Wochen lang in RPMI 1640 Medium, ergänzt mit 10 % fötalem Kälberserum, nicht essentiellen Aminosäuren, Antibiotika und 50 µM 2-Mercaptoethanol, bei 37 °C und 5 % CO₂ kultiviert. Die murinen Krebszelllinien B16, LLC und CT26 EpCam, und 11 humane Krebszelllinien aus dem NCI 60 Panel², sechs primäre humane Tumorzellpräparationen, und humane Rhabdomyosarkomzellen (A204 Zellen) wurden ebenfalls in komplettem RPMI 1640 Medium kultiviert. Die Zellen wurden, wenn nichts anderes angegeben ist, mit 100 ng/ml Maus- oder menschlichem Interferon-γ (R&D Systems, Wiesbaden, Deutschland), oder 10 ng/ml Maus- oder humanem TNF (R&D Systems) oder mit einer Kombination aus Maus- und humanem Interferon-γ (50-100 ng/ml) und Maus- oder humanem TNF (0,1-10 ng/ml) über 2 bis 6 Tage behandelt. Die β-Krebszellen wurden mittels Immunfluoreszenz unter Einsatz eines Anti-Synaptophysin-Antikörpers (unverdünnt; Lifespan Biosciences, Seattle, Washington, USA), identifiziert.

Knock-down von p16^{lnk4a}. 5 x 10⁴ β-Tumorzellen wurden in Zellkulturplatten ausgesät. Nach 72 h wurden die Zellen mit 2 ml Zellkulturüberstand, der shControl oder shp16/19 Mscv Vektoren enthielt, in Anwesenheit von 1 µg/ml Polybren (Sigma, München, Deutschland) für eine Gesamttransduktionszeit von 12 h transfiziert. Nach 5 Tagen wurden die transduzierten Zellen durch Behandlung mit 1 µg/ml Puromycin (Sigma) für 72 h selektioniert. Die Transduktionsrate wurde durch Zählen GFP-positiver Zellen unter einem Zeiss Axiovert 200 Mikroskop (Zeiss, Oberkochen, Germany) bestimmt und lag in der Regel bei >90%.

*In*-*vitro*-Proliferationsassays: Nach der Behandlung wie oben beschrieben, wurde die Proliferation der Krebszellen entweder mittels [³H]-Thymidin-Einbau oder durch einen BrdU-basierten Zellproliferations-ELISA oder durch den XTT-basierten Zellproliferationskit II gemäß den Angaben des Herstellers (Roche Diagnostics, Mannheim, Deutschland) gemessen.

*In*-*vitro*-Wachstums-Arrestassays: Die unterschiedlichen Krebszellen wurden mit einer Dichte von 1 x 10⁴ Zellen/cm² ausgesät. Anschließend wurden die Zellen entweder mit Kontrollmedium oder Zytokinen, wie weiter oben beschrieben, 4 bis 5 Tage lang behandelt. Nach der Behandlung wurden die Zellen trypsiniert und die lebenden Zellen mit einem Zeiss Axiovert 25 Mikroskop (Zeiss, Oberkochen, Deutschland) unter Einsatz einer Neubauer Zählkammer (Karl Hecht GmbH, Sondheim, Deutschland) ausgezählt. Die Zellen wurden mit 2 x 10⁴ Zellen/cm² wieder ausgesät und in komplettem RPMI 1640 Medium in Abwesenheit der Zytokine kultiviert bis die Kontrollkulturen Konfluenz erreichten. Anschließend wurden die Zellen trypsiniert, ausgezählt und wieder ausgesät (siehe auch Figur 13: Methode zum Screenen von Substanzen). Nach der Passage 1-2 wurden 1000 bis 3000 lebende Zellen auf Multiscreen™ HTS 96-Wellfiltrationsplatten (Millipore, Billerica, USA) ausgesät, und die Proliferation mittels des BrdU-basierten Zellproliferation-ELISA in Kombination mit dem Vector®SG Substratkit für Peroxidase (Vector Laboratories, Burlinggame, USA) gemessen, um die BrdU-einbauenden Zellen zu visualisieren. Die BrdU-positiven Spots wurden mit einem ELISPOT-Leser (BIO-SYS, Karben, Deutschland) ausgezählt.

Behandlung von Mäusen mit Tag- T_{H}1-Zellen: Vor der ersten Tag-T_{H}1-zellbasierten Therapie wurden alle Mäuse bestrahlt. Anschließend wurden 1 x 10⁷ Tag- T_{H}1-Zellen in 0,9 % NaCl-Lösung (Tag- T_{H}1) oder NaCI-Lösung alleine (Sham) intraperitoneal einmal pro Woche (Beginn: Lebenswoche 6) injiziert. Im Allgemeinen wurden die Mäuse nach 6 Wochen Behandlung (Lebenswoche 12) getötet.

Transfer von β-Krebszellen in Immun-defiziente Mäuse: β-Krebszellen, die aus verschiedenen Mäusegruppen isoliert wurden, wurden über 3 Passagen kultiviert. Anschließend wurden 10 bis 60 % der β-Krebszellen in immundefiziente NOD-SCIDxIL2Rcγ^{-/-}-Mäuse subkutan injiziert. Das Tumorwachstum wurde mit einem Messstab verfolgt und die Blutglucosewerte mit einem Accu-Check-Sensor (Roche Diagnostics) 7 Wochen lang gemessen.

Immunfluoreszenz und Immunhistochemie zur Untersuchung der Seneszenz und Redifferenzierung: Die unterschiedlichen Krebszellen wurden auf Kammerobjektträger kultiviert (BD Biosciences, Heidelberg, Deutschland). Nach der Behandlung wurden die Zellen mit Aceton/Methanol 1:1 fixiert und die Objektträger mit PBS/0.05 % Tween 20 bei Raumtemperatur gewaschen, mit serumfreien DAKO-Block (DAKO, Hamburg, Deutschland) blockiert, wieder gewaschen und anschließend mit den folgenden Antikörpern inkubiert: Anti-pHP1γ (Verdünnung 1:100; Abcam), anti-Ki67 (Verdünnung 1:100; Abcam), anti-p16Ink4a (Verdünnung 1:100; Santa Cruz Biotechnologie) oder anti-Glucosetransporter2 (anti-Glut2; Verdünnung 1:1000; Abcam). Nach einem Waschschritt wurden die Objektträger mit Anti-Kaninchen-Alexa488 (Invitrogen), Anti-Kaninchen-Cy3 (Dianova, Hamburg, Deutschland), Anti-Maus-Alexa555 oder Anti-Maus-Alexa488 (Cell Signaling Technology) inkubiert, wiederum gewaschen und anschließend mit DAPI (Invitrogen) inkubiert. Die Analyse erfolgte unter Einsatz eines Zeiss Axiovert 200 Mikroskops und der Visiview Software (Visitron Systems, Puchheim, Deutschland).

SA-ß-Galactosidase-Aktivitäts-Assay: Die Krebszellen wurden 15 min lang bei Raumtemperatur fixiert und anschließend 16 Stunden bei 37 °C unter Verwendung des β-Galactosidase-Färbekits (US Biological; Swampscott, USA) gefärbt. SA-β-gal-positive und -negative Zellen wurden unter Einsatz eines Zeiss Axiovert 200 Mikroskops ausgezählt.

Zellzyklusanalyse: Nach Behandlung der β-Krebszellen wurde die Zellzyklusanalyse mittels des BD Pharmingen FITC-BrdU Flow Kits gemäß den Anleitungen des Herstellers (BD Biosciences) durchgeführt. Die Proben wurden mittels Durchflusszytometrie auf einem LSR II von Becton Dickinson (Heidelberg, Deutschland) analysiert und die nachfolgenden Zellzyklusphasen in % der Gesamtpopulation bestimmt: subG₁ (apoptotische Zellen), G₁/G₀ (2n, BrdU-negativ), S (2n-4n, BrdU-positiv) und G₂/M-Phase (4n, BrdU-negativ).

Western Blot zur Untersuchung der Seneszenz und Redifferenzierung: Nach der Behandlung wurden die Krebszellen in Lysepuffer (50 mM Tris-HCl, pH 7.5, 150 mM NaCl, 1% Triton X-100, 0.5% SDS, 1mM NaF, 1mM Na₃VO₄, und 0.4% β-Mercaptoethanol) aufgeschlossen, der einen Proteaseinhibitorcocktail und einen Phosphataseinhibitorcocktail (PhosSTOP von Roche Diagnostics) enthält. Nach einer Proteinbestimmung mit dem Bicinchoninic-Acid-Assay (BCA; Thermo Fisher Scientific) wurden die Proteine durch eine 12% SDS-PAGE oder mittels vorgefertigten Mini Protean TGX Precast Gelen (4-15%; von BioRad) getrennt, auf eine PVDF Membran überführt und mit 3% Milchpulver in TBS/0.1% Tween 20 (TBST) geblockt. Die Membran wurde dann mit einem anti-Glucosetransporter2 (anti-Glut2; Verdünnung 1:1000; Abcam), Anti-Rb (Ab-780) (1:1000), Anti-Rb(phospho-Ser-795) (1:1000; beide Antikörper von SAB Signalway Antibody, Pearland, TX, USA) oder anti-β-Aktin Antikörper (1:1000; BioVision) inkubiert. Nach einem Waschschritt mit TBST und anschließendem Blockieren unspezifischer Bindungsstellen wurden die Blots mit einem Ziege anti-Kaninchen-Meerrettich-peroxidase (HRP)-konjugierten Antikörper (1:3000; Cell Signaling Technology) inkubiert und danach wieder gewaschen. Schließlich wurde die Antikörperbindung mit dem ECL Detektionsreagenz (Amersham) sichtbar gemacht.

### Array Comparative Genomische Hybridisierung (Array-CGH)

Mittels Comparativer Genomischer Hybridisierung (CGH) können genomweit quantitative chromosomale Aberrationen dargestellt werden, wie man sie bei soliden Tumoren im Rahmen der Tumorprogression und der damit einhergehenden genomischen Instabilität häufig findet.

Dazu wurde die DNA aus dem Tumorgewebe isoliert und mit einem Fluoreszenzmarker markiert. Parallel dazu wurde eine Kontroll-DNA von einem gesunden Spender mit einem zweiten Fluoreszenzmarker markiert. Beide DNAs wurden dann auf einen genomweiten Array von 105.000 Oligonucleotiden hybridisiert. Diese haben nun proportional zu ihrem relativen Gehalt in der Hybridisierungslösung gebunden. Aus dem Bindungsverhalten liess sich genomweit der DNA-Gehalt des Tumors relativ zum gesunden Genom errechnen und grafisch darstellen. Für die Analyse wurde der Agilent Human Genome CGH 105A Micro-Array (Agilent Technologies, Böblingen, Deutschland) verwendet und an einem DNA Microarray Scanner (Agilent Technologies) vermessen. Die erhaltenen Daten wurden mit der Software Feature Extraction 10.5.1.1 und DNA Analytics 4.0.85 (Agilent Technologies) auf der Grundlage des Human Genome Build 18 ausgewertet.

### Heilversuch

Im Rahmen eines Heilversuches wurde ein Patient auf eigenen Wunsch nach vollständiger Aufklärung mit einer Kombination von IFN-α und TNF behandelt. Der Patient hatte ein Peritonealkarzinom (Primärtumor: Melanom) mit starkem Aszites. Seine Überlebensprognose wurde auf 1-2 Wochen geschätzt. Die Behandlung erfolgte durch Aszitespunktion und Gabe von IFN-α und TNF. Bei der Überwachung wurde festgestellt, dass der Patient bereits endogen TNF in ausreichenden Mengen bildete, so dass in diesem Fall lediglich IFN-α gegeben werden musste.

### Ergebnisse

Fig. 1 ist der Nachweis zu entnehmen, dass die Kombination aus Substanzen, die den STAT1- und TNF-Signalweg aktivieren (hier: Interferon-γ und TNF) einen stabilen Wachstumsarrest von β-Krebszellen in vitro induzieren können. So ist in Fig. 1a eine Zellzyklusanalyse und das mittlere G₁/S-Verhältnis von β-Krebszellen dargestellt, die entweder in Gegenwart oder in Abwesenheit von Interferon-y und TNF kultiviert wurden. In Fig. 1b ist die Zellanzahl der lebenden Zellen von β-Krebszellen dargestellt, die 5 Tage lang mit Medium oder mit Interferon-γ und TNF behandelt wurden. Auch nach Entzug der Wachstums-inhibierenden Signale/Zytokine, die zur Aktivierung der STAT1 und TNFR1 Signalkaskade führten, verblieben die Zellen weiter stabil arretiert in der Seneszenz (Fig. 1B Periode nach dem Waschen).

Zu erkennen ist, dass die kombinierte Behandlung bewirkte, dass frisch isolierte β-Krebszellen in Gegenwart von Interferon-y und TNF in ihrem Wachstum vollständig arretiert wurden, wohingegen die unbehandelten Zellen in dem Medium schnell proliferierten. Der Zellzyklusanalyse ist auch zu entnehmen, dass sich die unbehandelten β-Krebszellen zu mehr als 25 % in der S-Phase und zu 40 % in der G₁/G₀-Phase befanden, was deren schnelle Proliferation erklärt. Die gemeinsame Aktivierung von STAT1 und TNFR1/CD95 bewirkte somit den Arrest der β-Krebszellen in der G₁/G₀-Phase innerhalb von 3 Tagen (siehe Fig. 1a). Nach 5 Tagen war dann der Wachstumsarrest von Dauer (Fig. 1b).

Fig. 2 ist zu entnehmen, dass die erfindungsgemäße Kombination aus STAT1 und TNFR1 aktivierenden Signalen, hier Interferon-y und TNF, auch die charakteristischen, Seneszenz-assoziierten Marker induziert, was hier durch den Nachweis der Seneszenz-assoziierten β-Galactosidase (SA-β-gal)-Aktivität dargestellt ist. In den Versuchen zeigte sich, dass innerhalb von 3 Tagen die erfindungsgemäße Kombination den Früh-Seneszenz-Marker pHP1γ in 75 % der β-Krebszellen und SA-β-gal in 50 % der Krebszellen induzierte (nicht gezeigt). Nach 4 Tagen schließlich wurde ein stabiler Wachstumsarrest induziert, was durch den Spät-Seneszenz-Marker SA-β-gal in 80 % der Zellen gezeigt werden konnte (siehe Fig. 2).

Fig. 3 zeigt die Ergebnisse der Untersuchung der Zellproliferation nach BrdU-Färbung, wobei vier verschiedene Versuchsansätze gezeigt sind: Wie weiter oben erläutert, wurden die β-Krebszellen 4 bis 5 Tage entweder mit Medium (CO.), nur STAT1-aktivierenden (Interferon-γ), nur TNFR1-aktivierenden (TNF), oder der Kombination aus STAT1- und TNFR1/CD95-aktivierenden Signalen (Interferon-γ und TNF) behandelt. Nach der Inkubation wurden die Zellen gewaschen, trypsiniert und anschließend in Abwesenheit der Zytokine zwei weitere Passagen kultiviert. Nach Passage 2 wurden 3000 lebende Zellen in 96 Wellplatten ausgesät und die Zellproliferation mittels einer BrdU-Färbung analysiert. Zu entnehmen ist, dass nur eine Kombination aus STAT1- und TNFR1-aktivierenden Signalen (Interferon-γ und TNF), jedoch weder die alleinige Aktivierung von STAT1 (Interferon-γ), noch die alleinige Aktivierung von TNFR1 (TNF), *in vitro* einen stabilen Wachstumsarrest in isolierten β-Krebszellen induzieren konnten. Fig. 3b zeigt die Mittelwerte der BrdU-positiven Spots der β-Krebszellen nach der Behandlung mit Medium, zwei Wochen nach der Induktionstherapie mit Interferon-γ, TNF oder der Kombination aus Interferon-γ und TNF.

Die in Fig. 2 dargestellten Ergebnisse wurden mit den in Fig. 4a und 4b dargestellten Ergebnissen weiter untermauert, wonach in entweder STAT1- oder TNFR1-defizienten β-Krebszellen keine Seneszenz-Induktion durch die kombinierte Applikation von Interferon-y und TNF erfolgte, was weiter bestätigt, dass die beiden Signalketten STAT1 und TNFR1 essentiell sind, bzw. deren Aktivierung essentiell ist, um die Seneszenz von Tumorzellen zu induzieren. Die Positiv-Kontrolle für 4a ist oben, das Experiment ist unten in 4a abgebildet und zeigt, dass auch die Kombination aus Interferon-y und TNF in TNFR1-defizienten Tumoren keine Seneszenz induziert.

Fig. 5a und 5b schließlich zeigen die Induktion der SA-β-gal-Aktivität und den mittleren Prozentsatz an seneszenten pHP1γ⁺-Zellen in Brustkrebszellen, die aus transgenen Mäusen isoliert wurden, 72 Stunden nach Behandlung mit Medium oder mit Interferon-γ und TNF. Auch hier zeigte sich, dass bei einer Behandlung mit der erfindungsgemäßen Kombination die beiden Seneszenz-Marker (β-Galactose-Aktivität und pHP1γ) im Vergleich zu den unbehandelten Zellen stark zugenommen haben. Fig. 5c zeigt beispielhaft die Veränderung des Phosphorylierungszustandes des Zellzyklusregulators Rb in Rhabdomyosarkomzellen des Menschen, der durch die IFN-γ und TNF Behandlung wieder normalisiert wird.

Fig 6a zeigt, dass short hairpin p16/19 (shp16/19) RNA p16^{lnk4a} herunterreguliert.

Fig. 6b zeigt, dass Zellen, die mit Kontroll-shRNA transduziert wurden, normal auf die Behandlung mit IFN-γ und TNF reagierten und einen Seneszenz-definierenden stabilen Zellzyklusarrest einleiteten (linkes Diagramm). Im Gegensatz dazu konnte nach einer Suppression von p16 mittels p16/19shRNA die Behandlung mit IFN-γ- und TNF in den Tumor-Zellen keinen Zellzyklusarrest mehr induzieren. Die Zellen proliferierten ungehindert schnell in der Gegenwart von IFN-γ- und TNF (rechtes Diagramm). Dies zeigt, dass p16^{lnk4a} für die IFN-γ- und TNF-induzierte Seneszenz unabdingbar ist. In Fig. 7 wurden β-Krebszellen mit Kontroll-shRNA oder shp16/19 Mscv Vektoren transduziert, und dann fünf Tage mit Medium oder mit IFN-γ und TNF behandelt. Danach wurden die Zellen gewaschen, trypsiniert und dann in Abwesenheit der Zytokine weiterkultiviert. Der Mittelwert der Zellzahlen lebender Zellen aus drei unabhängigen Kulturen ist bei den verschiedenen Passagen p-1 bis p1 angegeben.

Fig. 7 zeigt Tumorvolumen in immundefizienten NOD-SCIDxIL2Rcγ^{-/-} Mäusen nach Transfer von 10⁵ β-Krebszellen, die aus entweder Placebo- (sham) oder Tag-TH1 Zellbehandelten Mäusen stammen. In der Tabelle werden der Ursprung (aus normalen RIP1-Tag2 Mäusen links oder aus TNFR1-defizienten RIP1-Tag2 Mäusen rechts) und die Prozentzahl der aus der Kultur transferierten β-Krebszellen/Maus angegeben, die jeweils injiziert wurde. Da die TNFR1-/-xRIP1-Tag2 Zellen *in vitro* schneller proliferierten, wurden hier nur 10% der isolierten und *in vitro* erhaltenen β-Krebszellen transferiert. Fig. 7 zeigt, dass die *in vivo* über TNF- und IFN-γ-produzierende T_{H}1-Lymphozyten vermittelte Seneszenz, erst *in vitro* über drei Passagen stabil bleibt und dann selbst nach einem Transfer der seneszenten β-Krebszellen weiter *in vivo* über mindestens 6 Wochen anhält, da exklusiv in dieser Gruppe keine nachweisbaren Tumoren in immunsupprimierten Mäusen heranwachsen. Dahingegen wachsen die β-Krebszellen von Placebo-behandelten Mäusen zu großen Tumoren heran. Die Tumoren wuchsen insbesondere dann schnell und wurden sehr groß, wenn die Tumorzellen aus TNFR1^{-/-}xRIP1-Tag2 Mäusen isoliert wurden. Hier hatte auch die Therapie mit T_{H}1-Zellen keine Wirkung, was wiederum die Abhängigkeit der Seneszenzinduktion vom TNFR1 Signalweg auch *in vivo* belegt.

Figur 8 A demonstriert den Wachstumsarrest der Tumorzellen nach Beginn der T_{H}1-Behandlung (IFN-γ und TNF-produzierende Zellen) für insgesamt 17 Wochen (119 Tage). Es ist kein Tumorwachstum nach Transfer in immundefiziente Mäuse zu beobachten.

Figur 8 B zeigt den Wachstumsarrest der Melanomzelllinie WM115 in vitro nach Beginn der IFN-γ- und TNF-Behandlung: Die Zellen bleiben nach insgesamt 10 Passagen (das heisst > 45 Tage) stabil

Fig. 9 zeigt, dass die Adenom-ähnlichen Tumoren von Tag-T_{H}1-Zell-behandelten RIP1-Tag2 Mäusen genomisch stabil bleiben (Fig. 9A, blaue Linie: CGH-Analyse ohne Aberrationen im Genom der Tumoren aus RIP1-Tag2 Mäusen, die mit Tumor-spezifischen T_{H}1 Lymphozyten behandelt wurden). Dahingegen zeigt die CGH-Analyse der β-Zelltumoren von Placebo-behandelten Mäusen mehrere größere genomische Abweichungen, wie z. B. in Fig. 9B: einen Verlust der Chromosomen 8 und 9 und Teilen von Chromosom 10 (siehe schwarze Pfeile). Das X Chromosom wird als Positivkontrolle verwendet.

Tabelle 1 in Fig. 10 zeigt, dass verschiedene Mauskrebszelllinien durch IFN-γ und TNF Behandlung in die Seneszenz, das heißt in einen permanenten Wachstumsarrest, der auch nach Beendigung der eigentlichen Behandlung bestehen bleibt, getrieben werden. Weiterhin ist der Tabelle zu entnehmen, dass für die Induktion des permanenten Wachstumsarrests die gleichzeitige Aktivierung der STAT1- und der TNFR1-Signalkaskade benötigt wurde.

Zur Gewinnung der Daten der Tabelle 1 wurde die Expression der IFN-γ Rezeptoren IFNGR1 und IFNGR2, und des TNF Rezeptors TNFRSF1 in unbehandelten Krebszelllinien durch RT-PCR bestimmt. Der antiproliferative Effekt wurde durch Zellzahlbestimmung der lebenden Zellen bei p0 nach 4-5 Tagen IFN-γ und TNF Behandlung gemessen. Der permanente Wachstumsarrest wurde durch Zellzahlbestimmung der lebenden Zellen an p1, 3-4 Tage nach Wegnahme von IFN-γ und TNF und Expansion in Medium gemessen. Die Daten werden als Mittelwert ± S.E.M. von drei unabhängigen Kulturen angegeben (siehe Tabelle 1 in Fig. 9: Grau = Responder; Weiß = Non-Responder; * durch Messung der SA-β Gal bestimmt). Die Non-Responder haben entweder einen nachgewiesenen Defekt in der STAT1- oder in der TNFR1-Signaltransduktion.

Tabelle 2 in Fig. 11 zeigt, dass auch verschiedene humane Krebszelllinien durch IFN-γ und TNF Behandlung in die Seneszenz, das heißt in einen permanenten Wachstumsarrest, der auch nach Beendigung der eigentlichen Behandlung bestehen bleibt, überführt werden konnten. Zur Gewinnung der Daten in Tabelle 2 wurde die Expression der IFN-γ-Rezeptoren IFNGR1 und IFNGR2, und des TNF Rezeptors TNFRSF1 in unbehandelten Krebszelllinien durch RT-PCR bestimmt. Der antiproliferative Effekt wurde durch Zellzahlbestimmung der lebenden Zellen bei p0 nach 4 Tagen IFN-γ und TNF Behandlung gemessen. Der permanente Wachstumsarrest wurde durch Zellzahlbestimmung der lebenden Zellen bei p1, 3-4 Tage nach IFN-γ und TNF Wegnahme und Kultur mit reinem Medium gemessen. Die Daten werden als Mittelwert ± S.E.M. von drei unabhängigen Kulturen angegeben.

Tabelle 3 in Fig. 12 zeigt, dass auch verschiedene frisch isolierte Malignome/Tumoren des Menschen durch IFN-γ und TNF Behandlung in die Seneszenz, das heißt in einen permanenten Wachstumsarrest, der auch nach Beendigung der eigentlichen Behandlung bestehen bleibt, überführt werden konnten. Zur Gewinnung der Daten in Tabelle 3 wurden frisch isolierte Melanome und Sarkome verwendet: Zwei humane Rhabdomyosarkom- und 4 humane Melanom-Tumorzellpräparationen wurden direkt aus Patienten isoliert. Die Isolierung der Tumorzellen aus Patientenmaterial wurde vom lokalen Ethikkomitee genehmigt. Die Zellkultur erfolgte wie weiter oben unter Methoden angegeben.

Der antiproliferative Effekt auf primäre humane Tumorzellen (Melanome TüMel75, TüMel74H, ZüMel1H, oder ZüMel, und Sarkome SRH, ZCRH) wurde gemessen, indem lebende Zellen nach 4 (Melanomzellen) oder 12 Tagen (Rhabdomyosarkomzellen) IFN-γ- und TNF-Behandlung gezählt wurden. SRH, TüMel75, TüMel74H, ZüMel1H, oder ZüMel1 wurden jeweils mit 100 ng/ml IFN-γ und 10 ng/ml TNF behandelt. ZCRH Tumorzellen wurden mit 10 ng/ml IFN-γ und 1 ng/ml TNF behandelt. Der Wachstumsarrest wurde bestimmt, indem lebende Zellen nach 4 (Melanomzellen) oder 10 Tagen (Rhabdomyosarkomzellen) IFN-γ- und TNF-Entzug gezählt wurden. Die Daten zeigen ein repräsentatives Experiment von 3 unabhängigen Experimenten (SRH und ZCRH) oder den Mittelwert ± S.E.M. von drei unabhängigen Kulturen (TüMel75, TüMel74H, ZüMel1H, und ZüMel1; siehe Tabelle 3 in Fig. 11: Grau = Responder; weiß = Non-Responder). Bei den Tumorzellen ZCRH und TüMel74H war der stabile Wachstumsarrest mit zytotoxischen Effekten kombiniert.

Fig. 13 zeigt, dass isolierte, gänzlich entdifferenzierte β-Krebs-Zellen in Anwesenheit der T_{H}1-Zytokine IFN-γ und TNF wieder redifferenzieren. Dies wird daran gezeigt, dass entdifferenzierte β-Krebszellen nach der Therapie sogar wieder den späten β-Zell-Differenzierungsmarker Glucosetransporter2 (Glut2) exprimieren und dadurch wieder in funktionelle Inselzellen zurückgeführt wurden. Dies wurde sowohl durch Immunfluoreszenz (Fig. 13a) als auch durch Western Blot (Fig. 13b) nachgewiesen. Zellkerne sind blau dargestellt (Fig. 13a) und β-Aktin wurde als Ladungskontrolle für den Western Blot verwendet (Fig 13b).

Fig. 14 zeigt ein Schema eines Ausführungsbeispiels (die angegebenen Zellzahlen stellen Beispiele dar, sind aber nicht bindend), mittels dessen die Seneszenzinduktion eindeutig nachgewiesen werden kann. Die Krebszellen werden bei Passage -1 in einer definierten Zelldichte ausgesät und dann für 4-14 Tage mit einer Wirkstoffkombination behandelt. Danach werden die Krebszellen geerntet und gezählt, und in der gleichen Zelldichte in Abwesenheit der Wirkstoffe wieder ausgesät. Die Bestimmung des permanenten Wachstumsstops (Seneszenz) geschieht in Passage +1, ungefähr 4-14 Tage nach dem Wirkstoffentzug. Es sind die Versuchsbedingungen so anzupassen, dass die Kontroll-Zellpopulationen nicht konfluent werden und dadurch in einen Wachstumsarrest überführt werden. Durch automatisierte Zellzählung lässt sich diese Methode im High-Throughput-Verfahren anwenden.

Figur 15 a zeigt das Behandlungsschema und die Ergebnisse des Heilversuchs mit IFN-α und TNF. Zu den Zeitpunkten t₀, t₁, t₂ und t₃ wird Ascites abgenommen. Nach der ersten Abnahme (t₀) wird der Patient mit IFN-α behandelt. Zu den Zeitpunkten t₀, t₁ und t₃ werden adhärente Zellen aus der Ascitesflüssigkeit isoliert und auf Zellkulturplättchen wachsen gelassen.

Figur 15 b zeigt weitere Daten des gleichen Heilversuchs:
Zu den Zeitpunkten t₀, t₁ und t₃ wird der Seneszenzmarker p16^{lnk4a} und der Proliferationsmarker Ki67 per Immunfluoreszenzmikroskopie bestimmt. Während Ki67 abnimmt, kommt es zu einer starken Induktion von p16^{lnk4a} nach *in vivo* Zytokinbehandlung.

Zum Zeitpunkt der vorliegenden Anmeldung lebt der Patient bereits mehr als 6 Monate (ursprüngliche Überlebensprognose 1-2 Wochen) in relativ stabilem Zustand. Die Mobilität hat sich auf 90% erhöht (von ursprünglich 30%), er kann wieder spazieren gehen (ursprünglich war er bettlägerig).

Figur 16: (A) IFN-α plus TNF induzieren Seneszenz in primären Tumorzellen eines Patienten mit Peritonealkarzinose: Insbesondere die Behandlung mit IFN-α plus TNF führt zu deutlich weniger Tumorzellen verglichen mit der Kontrolle (Ko.), die zudem eine Neuriten-artige (Pfeile) oder Spiegelei-artige (gestrichelte Linie) Morphologie annehmen. (B) IFN-α plus TNF-behandelte Tumorzellen sind in ihrer Proliferation stark gehemmt. (C) Die Aktivität der Seneszenz-assoziierten β-Galaktosidase (SA-β-Gal.) steigt nach IFN-α plus TNF auf über 70 % an. (D) Die entsprechenden Tumorzellen zeigen eine sehr starke Expression des Seneszenzmarkers p16^{lnk4a}.

## Patentansprüche

1. Kombination aus TNF-α und IFN-γ zur Anwendung in einem Verfahren für die Behandlung von Tumoren durch Induktion eines dauerhaften Wachstumsarrests (Seneszenz) in Tumorzellen, bei welchen p16^{Ink4a} präsent ist,
wobei die Substanzen jeweils in einer Konzentration von 0,0001 ng/ml bis 10.000 ng/ml in der pharmazeutischen Zusammensetzung am Wirkort eingesetzt werden und wobei die Verwendung der Kombination über einen Zeitraum von mindestens 3-4 Tage bis maximal 30 Tagen erfolgt.

2. Kombination aus TNF-α und IFN-γ zur Anwendung in einem Verfahren für die Behandlung von Tumoren durch Induktion eines dauerhaften Wachstumsarrests (Seneszenz) in Tumorzellen, bei welchen p16^{Ink4a} präsent ist gemäß Anspruch 1, wobei die Kombination 10 ng/ml TNF-α und 100 ng/ml IFN-γ
oder 1 ng/ml TNF-α und 10 ng/ml IFN-γ enthält.

3. Kombination aus TNF-α und IFN-γ zur Anwendung in einem Verfahren für die Behandlung von Tumoren durch Induktion eines dauerhaften Wachstumsarrests (Seneszenz) in Tumorzellen, bei welchen p16^{Ink4a} präsent ist, gemäß mindestens einem der Ansprüche 1 bis 2,
wobei es sich bei den Tumorzellen um prämaligne oder maligne, bona fide Tumorzellen/Tumoren oder Tumorstammzellen handelt.

4. Kombination aus TNF-α und IFN-γ zur Anwendung in einem Verfahren für die Behandlung von Tumoren durch Induktion eines dauerhaften Wachstumsarrests (Seneszenz) in Tumorzellen, bei welchen p16^{lnk4a} präsent ist gemäß mindestens einem der Ansprüche 1 bis 3,
wobei der Tumor ausgewählt ist aus HPV-induzierten benignen Tumoren, Präkanzerosen, Karzinomen, insbesondere Karzinomen der Haut, Schleimhäute, Lunge, Prostata, Brust, Ovarien, Sarkome, Melanome, ZNS-Tumoren oder Lymphomen/Leukämien.

5. Kombination aus TNF-α und IFN-γ zur Anwendung in einem Verfahren für die Behandlung von Tumoren durch Induktion eines dauerhaften Wachstumsarrests (Seneszenz) in Tumorzellen, bei welchen p16^{lnk4a} präsent ist gemäß mindestens einem der Ansprüche 1 bis 4, wobei die Verwendung alle 3 bis 6 Wochen für eine Gesamtdauer von zwei Jahren erfolgt.

6. Kombination aus TNF-α und IFN-γ zur Anwendung in einem Verfahren für die Behandlung von Tumoren durch Induktion eines dauerhaften Wachstumsarrests (Seneszenz) in Tumorzellen, bei welchen p16^{lnk4a} präsent ist, gemäß gemäß mindestens einem der Ansprüche 1 bis 5,
wobei die Applikation topisch, intratumoral, peritumoral, systemisch oder perkutan erfolgt.

## Claims

1. A combination of TNF-α and IFN-γ for use in a method for the treatment of tumors by induction of a permanent growth arrest (senescence) in tumor cells, in which p16^{lnk4a} is present,
wherein the substances are each employed in a concentration of 0.0001 ng/ml to 10,000 ng/ml in the pharmaceutical composition at the site of action and wherein the use of the combination is performed over a period of time of at least 3 to 4 days to maximum 30 days.

2. The combination of TNF-α and IFN-γ for use in a method for the treatment of tumors by induction of a permanent growth arrest (senescence) in tumor cells, in which p16^{lnk4a} is present, according to claim 1,
wherein the combination contains 10 ng/ml TNF-α and 100 ng/ml IFN-γ or 1 ng/ml TNF-α and 10 ng/ml IFN-γ.

3. The combination of TNF-α and IFN-γ for use in a method for the treatment of tumors by induction of a permanent growth arrest (senescence) in tumor cells, in which p16^{lnk4a} is present, according to at least one of claims 1 to 2,
wherein the tumor cells are premalignant or malignant, bona fide tumor stem cells/tumors, or tumor stem cells.

4. The combination of TNF-α and IFN-γ for use in a method for the treatment of tumors by induction of a permanent growth arrest (senescence) in tumor cells, in which p16^{lnk4a} is present, according to at least one of claims 1 to 3,
wherein the tumor is selected from HPV-induced benign tumors, precanceroses, carcinomas, in particular carcinomas of the skin, mucous membranes, lung, prostate, breast, ovaries, sarcomas, melanomas, CNS tumors or lymphomas/leukemias.

5. The combination of TNF-α and IFN-γ for use in a method for the treatment of tumors by induction of a permanent growth arrest (senescence) in tumor cells, in which p16^{lnk4a} is present, according to at least one of claims 1 to 4,
wherein the use is performed every 3 to 6 weeks for a total duration of two years.

6. The combination of TNF-α and IFN-γ for use in a method for the treatment of tumors by induction of a permanent growth arrest (senescence) in tumor cells, in which p16^{lnk4a} is present, according to at least one of claims 1 to 5,
wherein the application is performed topically, intratumorally, peritumorally, systemically, or percutaneously.

## Revendications

1. Combinaison de TNF-α et IFN-γ utile dans un procédé pour le traitement de tumeurs par induction d'un arrêt de croissance permanent (sénescence) dans des cellules de cancer, dans lesquelles p16^{lnk4a} est présent,
dans laquelle les substances sont chacune utilisées à une concentration de 0,0001 ng/ml à 10,000 ng/ml dans la composition pharmaceutique au lieu d'action et dans laquelle l'utilisation de la combinaison a lieu pendant une durée d'au moins de 3 à 4 jours jusqu'à 30 jours au maximum.

2. Combinaison de TNF-α et IFN-γ utile dans un procédé pour le traitement de tumeurs par induction d'un arrêt de croissance permanent (sénescence) dans des cellules de cancer, dans lesquelles p16^{lnk4a} est présent, selon la revendication 1,
dans laquelle la combinaison contient de 10 ng/ml TNF-α et 100 ng/ml IFN-γ ou 1 ng/ml TNF-α et 10 ng/ml IFN-γ.

3. Combinaison de TNF-α et IFN-γ utile dans un procédé pour le traitement de tumeurs par induction d'un arrêt de croissance permanent (sénescence) dans des cellules de cancer, dans lesquelles p16^{lnk4a} est présent, selon au moins une des revendications 1 à 2,
dans laquelle les cellules de cancer sont des cellules de cancer/tumeurs ou cellules souches de cancer bona fide prémalignes ou malignes.

4. Combinaison de TNF-α et IFN-γ utile dans un procédé pour le traitement de tumeurs par induction d'un arrêt de croissance permanent (sénescence) dans des cellules de cancer, dans lesquelles p16^{lnk4a} est présent, selon au moins une des revendications 1 à 3,
dans laquelle la tumeur est choisie à partir de tumeurs bénignes induites par VPH, précancéroses, carcinomes, en particulier des carcinomes de la peau, des muqueuses, du poumon, de la prostate, du sein, des ovaires, sarcomes, mélanomes, tumeurs du SNC ou lymphomes/leucémies.

5. Combinaison de TNF-α et IFN-γ utile dans un procédé pour le traitement de tumeurs par induction d'un arrêt de croissance permanent (sénescence) dans des cellules de cancer, dans lesquelles p16^{lnk4a} est présent, selon au moins une des revendications 1 à 4,
dans laquelle l'utilisation a lieu toutes les 3 à 6 semaines pour une durée totale de deux années.

6. Combinaison de TNF-α et IFN-γ utile dans un procédé pour le traitement de tumeurs par induction d'un arrêt de croissance permanent (sénescence) dans des cellules de cancer, dans lesquelles p16^{lnk4a} est présent, selon au moins une des revendications 1 à 5,
dans laquelle l'application se fait topiquement, intratumoralement, péritumoralement, systémiquement ou percutanément.
